(19) **Europäisches Patentamt / European Patent Office / Office européen des brevets**

(11) **EP 4 510 935 B1**

(12) **EUROPEAN PATENT SPECIFICATION**

(45) Date of publication and mention
of the grant of the patent:
**06.05.2026  Bulletin 2026/19**

(21) Application number: **23936794.9**

(22) Date of filing: **30.05.2023**

(51) International Patent Classification (IPC):
*A61B 6/03* (2006.01)   *G06T 5/00* (2024.01)
*A61B 6/00* (2024.01)   *G06T 11/00* (2026.01)
*G06T 5/60* (2024.01)   *G06T 5/70* (2024.01)

(52) Cooperative Patent Classification (CPC):
**G06T 5/70; A61B 6/037; A61B 6/486;
A61B 6/5205; G06T 5/60; G06T 12/20;**
G06T 2207/10104; G06T 2207/20081;
G06T 2207/20084; G06T 2211/412

(86) International application number:
**PCT/CN2023/097218**

(87) International publication number:
**WO 2024/243816 (05.12.2024 Gazette 2024/49)**

(54) **SYSTEMS AND METHODS FOR POSITRON EMISSION TOMOGRAPHY IMAGING**

SYSTEME UND VERFAHREN ZUR POSITRONENEMISSIONSTOMOGRAFIEBILDGEBUNG

SYSTÈMES ET PROCÉDÉS D'IMAGERIE PAR TOMOGRAPHIE À ÉMISSION DE POSITRONS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**26.02.2025  Bulletin 2025/09**

(73) Proprietor: **Shanghai United Imaging Healthcare
Co., Ltd.
Shanghai 201807 (CN)**

(72) Inventor: **LYU, Yang
Shanghai 201807 (CN)**

(74) Representative: **Wang, Bo
Panovision IP
Ebersberger Straße 3
85570 Markt Schwaben (DE)**

(56) References cited:
CN-A- 110 298 897   CN-A- 110 507 343
CN-A- 112 686 967   CN-A- 114 677 415
CN-A- 115 170 606   US-A1- 2013 261 440
US-A1- 2014 212 014   US-A1- 2020 090 378
US-A1- 2020 090 378   US-A1- 2020 320 753
US-A1- 2020 320 753   US-A1- 2021 304 457
US-A1- 2022 215 599   US-A1- 2023 080 348

## Description

### TECHNICAL FIELD

The present disclosure relates to medical imaging technology, and in particular, to systems and methods for positron emission tomography (PET) imaging.

### BACKGROUND

PET imaging has been widely used in clinical examination and disease diagnosis in recent years. In particular, dynamic PET imaging can provide a set of images over a dynamic scan time period and dynamic PET data can also provide rich information related to physiological parameters (e.g., perfusion pressure) that indicate the functional status of the imaged tissue(s) or organ(s). However, the dynamic PET imaging generally requires a relatively long scan time and a relatively long image reconstruction time, and also has a relatively low image quality. Therefore, it is desirable to provide systems and methods for PET imaging with improved imaging efficiency and image quality. Document US2013261440A1 may be considered to disclose the preamble of the independent claims.

### SUMMARY

According to an aspect of the present disclosure, a system for PET imaging may be provided. The system may include at least one storage device including a set of instructions for medical imaging and at least one processor in communication with the at least one storage device. When executing the set of instructions, the at least one processor may be configured to direct the system to perform the following operations. The system may obtain scan data of an object collected by a PET scan over a scan time period. The system may determine a plurality of target sets of scan data from the scan data based on a preset condition, wherein each of the plurality of target sets of scan data corresponds to a target sub-time period in the scan time period. The system may also generate one or more intermediate images corresponding to one or more sub-time periods different from the plurality of target sub-time periods in the scan time period based on the plurality of target sets of scan data. The system may further generate a target image sequence of the object based on the plurality of target sets of scan data and the one or more intermediate images.

In some embodiments, to determine a plurality of target sets of scan data from the scan data based on a preset condition, the system may perform the following operations. The system may divide the scan data into a plurality of candidate sets of scan data. For each of the plurality of candidate sets of scan data, the system may determine a three-dimensional (3D) counting distribution map corresponding to the candidate set of scan data. The 3D counting distribution map may include at least one pixel each of which corresponds to a pixel value indicating a count of coincidence events associated with the pixel. Further, the system may determine the plurality of target sets of scan data based on a plurality of 3D counting distribution maps corresponding to the plurality of candidate sets of scan data respectively.

In some embodiments, to determine the plurality of target sets of scan data based on a plurality of 3D counting distribution maps corresponding to the plurality of candidate sets of scan data respectively, the system may perform the following operations. The system may arrange the plurality of 3D counting distribution maps in chronological order to form a map sequence. Further, the system may determine the plurality of target sets of scan data by traversing the map sequence starting from the first 3D counting distribution map in the map sequence. The traversing the map sequence starting from the first 3D counting distribution map may include determining a difference between the latest 3D counting distribution map corresponding to the latest determined target set of scan data and each of the 3D counting distribution maps after the latest 3D counting distribution map in sequence until the difference between the latest 3D counting distribution map and one of the 3D counting distribution maps after the latest 3D counting distribution map is larger than or equal to a preset threshold, and designating a candidate set of scan data corresponding to the one of the 3D counting distribution maps as a target set of scan data.

In some embodiments, to determine a plurality of target sets of scan data from the scan data based on a preset condition, the system may perform the following operations. The system may obtain a time-activity curve associated with a tracer for the PET scan. The system may further determine the plurality of target sets of scan data from the scan data based on the time-activity curve.

In some embodiments, to determining a plurality of target sets of scan data from the scan data based on a preset condition, the system may perform the following operations. The system may obtain a vital signal of the object corresponding to the scan data. Further, the system may determine the plurality of target sets of scan data from the scan data based on the vital signal.

In some embodiments, to generate one or more intermediate images correspond to one or more sub-time periods different from the plurality of target sub-time periods in the scan time period, the system may generate a plurality of target images corresponding to the plurality of target sets of scan data respectively. Further, the system may generate the one or more intermediate images based on the plurality of target images.

In some embodiments, to generate a plurality of target images corresponding to the plurality of target sets of scan data respectively, the system may perform the following operations. The system may determine a plurality of preliminary images corresponding to the plurality of target sets of scan data respectively. Further, the system may determine the plurality of target images by performing a de-noise operation on the plurality of preliminary images using a de-noise model and/or performing a resolution-improve operation on the plurality of preliminary images using a resolution-improve model.

In some embodiments, to generate one or more intermediate images correspond to one or more sub-time periods different from the plurality of target sub-time periods in the scan time period, the system may perform the following operations. For each pair of one or more pairs of target images among the plurality of target images, the system may determine a motion field between the pair of target images using a motion field generation model. Further, the system may generate one or more intermediate images corresponding to the pair of target images based on the motion field using an image generation model.

In some embodiments, the motion field generation model and the image generation model may be integrated into a single model.

In some embodiments, to generate a target image sequence of the object based on the plurality of target sets of scan data and the one or more intermediate images, the system may perform the following operations. For each pair of one or more pairs of images among the plurality of target images and the one or more intermediate images, the system may determine a secondary motion field between the pair of images using a motion field generation model, and further generate one or more secondary intermediate images corresponding to the pair of images based on the secondary motion field using an image generation model. Further, the system may generate the target image sequence of the object based on the plurality of target images, the one or more intermediate images, one or more secondary intermediate images.

In some embodiments, a difference between the pair of images may be larger than a preset difference threshold.

According to another aspect of the present disclosure, a method for PET imaging may be provided. The method may be implemented on a computing device having at least one storage device and at least one processor. The method may include obtaining scan data of an object collected by a PET scan over a scan time period. The method may also include determining a plurality of target sets of scan data from the scan data based on a preset condition, wherein each of the plurality of target sets of scan data corresponds to a target sub-time period in the scan time period. The method may also include generating one or more intermediate images corresponding to one or more sub-time periods different from the plurality of target sub-time periods in the scan time period based on the plurality of target sets of scan data. The method may further include generating a target image sequence of the object based on the plurality of target sets of scan data and the one or more intermediate images.

According to yet another aspect of the present disclosure, a system for PET imaging may be provided. The system may include an obtaining module, a determination module, and a generation module. The obtaining module may be configured to obtain scan data of an object collected by a PET scan over a scan time period. The determination module may be configured to determine a plurality of target sets of scan data from the scan data based on a preset condition, wherein each of the plurality of target sets of scan data corresponds to a target sub-time period in the scan time period. The generation module may be configured to generate one or more intermediate images corresponding to one or more sub-time periods different from the plurality of target sub-time periods in the scan time period based on the plurality of target sets of scan data, and generate a target image sequence of the object based on the plurality of target sets of scan data and the one or more intermediate images.

According to yet another aspect of the present disclosure, a non-transitory computer readable medium may be provided. The non-transitory computer readable medium may include at least one set of instructions for PET imaging. When executed by one or more processors of a computing device, the at least one set of instructions causes the computing device to perform a method. The method may include obtaining scan data of an object collected by a PET scan over a scan time period. The method may also include determining a plurality of target sets of scan data from the scan data based on a preset condition, wherein each of the plurality of target sets of scan data corresponds to a target sub-time period in the scan time period. The method may also include generating one or more intermediate images corresponding to one or more sub-time periods different from the plurality of target sub-time periods in the scan time period based on the plurality of target sets of scan data. The method may further include generating a target image sequence of the object based on the plurality of target sets of scan data and the one or more intermediate images.

According to yet another aspect of the present disclosure, a device for PET imaging. The device may include at least one processor and at least one storage device for storing a set of instructions. When the set of instructions are executed by the at least one processor, the device performs the methods of the present disclosure.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

## BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings.

FIG. 1 is a schematic diagram illustrating an exemplary PET system according to some embodiments of the present disclosure.

FIG. 2 is a schematic diagram illustrating exemplary hardware and/or software components of an exemplary computing device according to some embodiments of the present disclosure.

FIG. 3 is a block diagram illustrating an exemplary processing device according to some embodiments of the present disclosure.

FIG. 4 is a flowchart illustrating an exemplary process for generating a target image sequence of an object during a dynamic PET imaging according to some embodiments of the present disclosure.

FIG. 5A is a flowchart illustrating an exemplary process for determining a plurality of target sets of scan data according to some embodiments of the present disclosure.

FIG. 5B is a schematic diagram illustrating exemplary 3D counting distribution maps according to some embodiments of the present disclosure.

FIG. 6 is a schematic diagram illustrating an exemplary TAC according to some embodiments of the present disclosure.

FIG. 7 is a schematic diagram illustrating an exemplary respiratory curve according to some embodiments of the present disclosure.

FIG. 8 is a schematic diagram illustrating an exemplary process for generating a plurality of target images according to some embodiments of the present disclosure.

FIG. 9 is a schematic diagram illustrating an exemplary process for generating a target image sequence according to some embodiments of the present disclosure.

FIG. 10A is a schematic diagram illustrating an exemplary target image sequence according to some embodiments of the present disclosure.

FIG. 10B is a schematic diagram illustrating another exemplary target image sequence according to some embodiments of the present disclosure.

## DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure. Various modifications to the disclosed embodiments will be readily apparent to those skilled in the art, and the general principles defined herein may be applied to other embodiments and applications without departing from the scope of the present disclosure. Thus, the present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

It will be understood that the term "system," "engine," "unit," "module," and/or "block" used herein are one method to distinguish different components, elements, parts, sections or assembly of different levels in ascending order. However, the terms may be displaced by another expression if they achieve the same purpose.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items.

The term "pixel" and "voxel" in the present disclosure are used interchangeably to refer to an element of an image. An anatomical structure shown in an image of a subject (e.g., a patient) may correspond to an actual anatomical structure existing in or on the subject's body. The term "object" and "subject" in the present disclosure are used interchangeably to refer to a biological object (e.g., a patient, an animal) or a non-biological object (e.g., a phantom). In some embodiments, the object may include a specific part, organ, and/or tissue of the object. For example, the object may include the head, the bladder, the brain, the neck, the torso, a shoulder, an arm, the thorax, the heart, the stomach, a blood vessel, soft tissue, a knee, a foot, or the like, or any combination thereof, of a patient.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

As aspect of the present disclosure provides systems and methods for PET imaging. The systems may obtain scan data of an object collected by a PET scan over a scan time period. The systems may also determine a plurality of target sets of scan data from the scan data. Each of the plurality of target sets of scan data may correspond to a target sub-time period in the scan time period. Further, the systems may generate one or more intermediate images corresponding to one or more sub-time periods different from the plurality of target sub-time periods in the scan time period based on the plurality of target sets of scan data. For example, the systems may generate a plurality of target images based on the plurality of target sets of scan data. For each pair of one or more pairs of target images among the plurality of target images, the systems may determine a motion field between the pair of target images using a motion field generation model and generate one or more intermediate images corresponding to the pair of target images based on the motion field using an image generation model. Then, the systems may generate a target image sequence of the object based on the plurality of target sets of scan data and the one or more intermediate images.

According to some embodiments of the present disclosure, intermediate image(s) may be generated (e.g., based on a motion field between a pair of target images) without image reconstruction, accordingly, an image reconstruction time can be reduced. Further, the intermediate image(s) may be generated according to a motion field generation model and an image generation model, and/or before the generation of the target images, a de-noise operation may be performed using a de-noise model and/or a resolution-improve operation may be performed using a resolution-improve model. Accordingly, the imaging efficiency and the image quality can be improved.

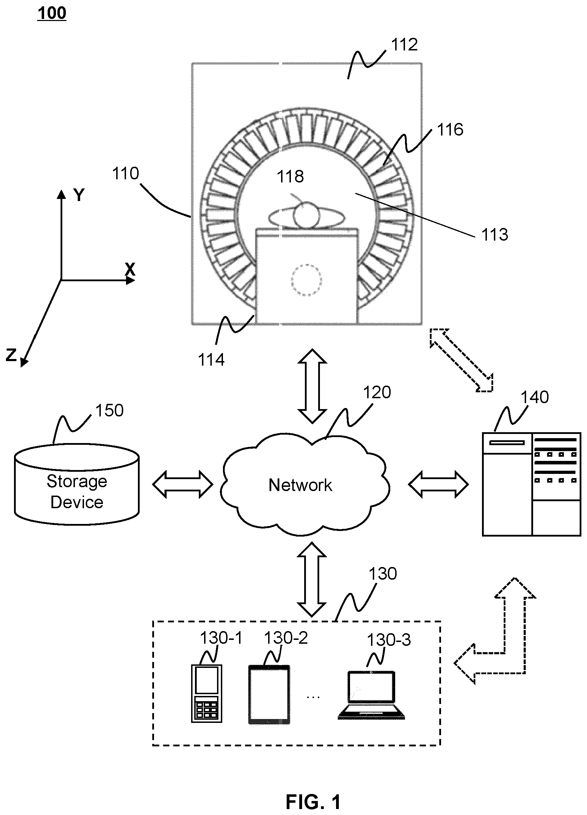

FIG. 1 is a schematic diagram illustrating an exemplary PET system according to some embodiments of the present disclosure. In some embodiments, as illustrated in FIG. 1, the PET system 100 may include a PET scanner 110, a network 120, a terminal device 130, a processing device 140, and a storage device 150. The components of the PET system 100 may be connected in various manners. Merely by way of example, the PET scanner 110 may be connected to the processing device 140 through the network 120. As another example, the PET scanner 110 may be connected to the processing device 140 directly as indicated by the bi-directional arrow in dotted line linking the PET scanner 110 and the processing device 140. As a further example, the processing device 140 may be connected to the storage device 150 through the network 120 or directly. As still a further example, the terminal device 130 may be connected to the processing device 140 through the network 120 or directly as indicated by the bi-directional arrow in dotted lines linking the terminal device 130 and the processing device 140.

The PET scanner 110 may be configured to acquire scan data relating to an object. For example, the PET scanner 110 may scan the object or a portion thereof that is located within its detection region and generate the scan data relating to the object or the portion thereof. In some embodiments, the PET scanner 110 may perform a dynamic PET imaging over a dynamic scan time period on the object or the portion thereof and acquire corresponding scan data. The dynamic scan time period may include multiple sub-time periods (which may be manually defined or automatically divided) each of which corresponds to a set of scan data.

In some embodiments, the PET scanner 110 may include a gantry 112, a couch 114, and a detector 116. The gantry 112 may support the detector 116. The couch 114 may be used to support an object 118 to be scanned. The detector 116 may include a plurality of detector rings arranged along an axial direction (e.g., Z-axis direction in FIG. 1) of the gantry 112. In some embodiments, a detector ring may include a plurality of detector units arranged along the circumference of the detector ring. In some embodiments, the detector 116 may include a scintillation detector (e.g., a cesium iodide detector), a gas detector, or the like, or any combination thereof.

In some embodiments, before a PET scanning, the object 118 may be injected with a tracer species. The tracer species may refer to a radioactive substance that decays and emits positrons. In some embodiments, the tracer species may be radioactively marked radiopharmaceutical, which is a drug having radioactivity and is administered to the object 118. For example, the tracer species may include fluorine-18 ($^{18}$F) fluorodeoxyglucose (FDG), etc. During the scanning, pairs of photons (e.g., gamma photons) may result from the annihilation of positrons originating from the tracer species in the object 118. A pair of photons may travel in opposite directions. At least a part of the pairs of photons may be detected and/or registered by the detector units in the detector 116. A coincidence event may be recorded when a pair of photons generated by the positron-electron annihilation are detected within a coincidence time window (e.g., within 6 to 12 nanoseconds). The coincidence event may be assumed to occur along a line connecting a pair of detector units, and the line may be called as a "line of response" (LOR). The detector 116 may obtain counts of coincidence events based on the LORs for detected coincidence events and time points at which the coincidence events occurred.

In some embodiments, the PET scanner 110 may also be a multi-modality scanner, for example, a positron emission tomography-magnetic resonance imaging (PET-MRI) system, a positron emission tomography-computed tomography (PET-CT) system, etc.

The network 120 may facilitate exchange of information and/or data. In some embodiments, one or more components (e.g., the PET scanner 110, the terminal device 130, the processing device 140, the storage device 150) of the PET system 100 may send information and/or data to other component(s) of the PET system 100 via the network 120. For example, the processing device 140 may obtain, via the network 120, scan data relating to the object 118 or a portion thereof from the PET scanner 110. In some embodiments, the network 120 may be any type of wired or wireless network, or a combination thereof.

The terminal device 130 may include a mobile device 130-1, a tablet computer 130-2, a laptop computer 130-3, or the like, or any combination thereof. In some embodiments, the terminal device 130 may remotely operate the PET scanner 110. In some embodiments, the terminal device 130 may operate the PET scanner 110 via a wireless connection. In some embodiments, the terminal device 130 may receive information and/or instructions inputted by a user, and send the received information and/or instructions to the PET scanner 110 or the processing device 140 via the network 120. In some embodiments, the terminal device 130 may receive data and/or information from the processing device 140. In some embodiments, the terminal device 130 may be part of the processing device 140. In some embodiments, the terminal device 130 may be omitted.

The processing device 140 may process data obtained from the PET scanner 110, the terminal device 130, the storage device 150, or other components of the PET system 100. For example, the processing device 140 may obtain scan data (e.g., PET data) of an object (e.g., a human body) over a scan time period from the PET scanner 110 or the storage device 150. The processing device 140 may determine a plurality of target sets of scan data from the scan data. Each of the plurality of target sets of scan data may correspond to a target sub-time period in the scan time period. Then the processing device 140 may generate one or more intermediate images corresponding to one or more sub-time periods different from the plurality of target sub-time periods in the scan time period based on the plurality of target sets of scan data. Further, the processing device 140 may generate a target image sequence of the object based on the plurality of target sets of scan data and the one or more intermediate images.

In some embodiments, the processing device 140 may be a central processing unit (CPU), a digital signal processor (DSP), a system on a chip (SoC), a microcontroller unit (MCU), or the like, or any combination thereof. In some embodiments, the processing device 140 may be a single server or a server group. In some embodiments, the processing device 140 may be local to or remote from the PET system 100. In some embodiments, the processing device 140 may be implemented on a cloud platform. Merely by way of example, the cloud platform may include a private cloud, a public cloud, a hybrid cloud, a community cloud, a distributed cloud, an inter-cloud, a multi-cloud, or the like, or any combination thereof.

Merely for illustration, only one processing device 140 is described in the medical system 100. However, it should be noted that the medical system 100 in the present disclosure may also include multiple processing devices. Thus operations and/or method steps that are performed by one processing device 140 as described in the present disclosure may also be jointly or separately performed by the multiple processing devices. For example, if in the present disclosure the processing device 140 of the medical system 100 executes both process A and process B, it should be understood that the process A and the process B may also be performed by two or more different processing devices jointly or separately in the medical system 100 (e.g., a first processing device executes process A and a second processing device executes process B, or the first and second processing devices jointly execute processes A and B).

The storage device 150 may store data, instructions, and/or any other information. In some embodiments, the storage device 150 may store data obtained from the processing device 140, the terminal device 130, and/or the PET scanner 110. For example, the storage device 150 may store scan data collected by the PET scanner 110. As another example, the storage device 150 may store the target image sequence of the object. In some embodiments, the storage device 150 may store data and/or instructions that the processing device 140 may execute or use to perform exemplary methods described in the present disclosure.

In some embodiments, the storage device 150 may include a mass storage, removable storage, a volatile read-and-write memory, a read-only memory (ROM), or the like, or any combination thereof. In some embodiments, the storage device 150 may be implemented on the cloud platform described elsewhere in the present disclosure. In some embodiments, the storage device 150 may be connected to the network 120 to communicate with one or more components (e.g., the PET scanner 110, the terminal device 130, the processing device 140) of the PET system 100. One or more components of the PET system 100 may access the data or instructions stored in the storage device 150 via the network 120. In some embodiments, the storage device 150 may be part of the processing device 140.

It should be noted that the above description of the PET system 100 is merely provided for the purposes of illustration, and not intended to limit the scope of the present disclosure. For persons having ordinary skills in the art, multiple variations and modifications may be made under the teachings of the present disclosure. For example, the PET system 100 may include one or more additional components and/or one or more components of the PET system 100 described above may be omitted. Additionally or alternatively, two or more components of the PET system 100 may be integrated into a single component. A component of the PET system 100 may be implemented on two or more sub-components.

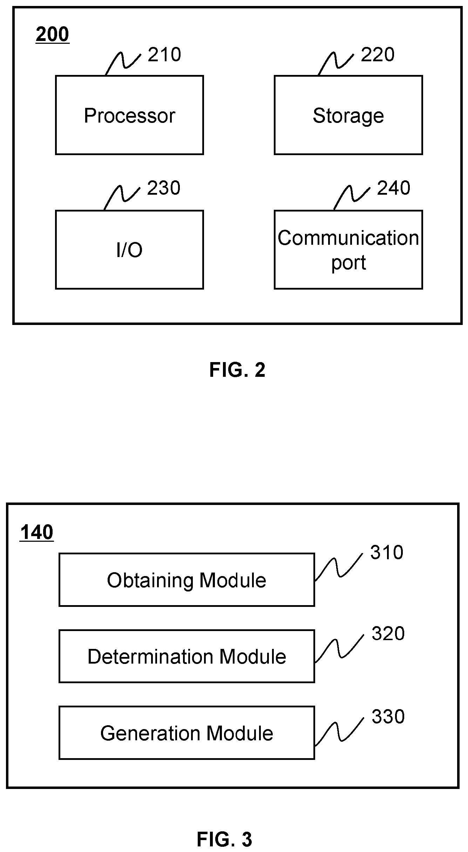

FIG. 2 is a schematic diagram illustrating exemplary hardware and/or software components of an exemplary computing device according to some embodiments of the present disclosure. In some embodiments, the processing device 140 may be implemented on the computing device 200. As illustrated in FIG. 2, the computing device 200 may include a processor 210, a storage 220, an input/output (I/O) 230, and a communication port 240.

The processor 210 may execute computer instructions (program code) and perform functions of the processing device 140 in accordance with techniques described herein. The computer instructions may include routines, programs, objects, components, signals, data structures, procedures, modules, and functions, which perform particular functions described herein. Merely for illustration purposes, only one processor is described in the computing device 200. However, it should be noted that the computing device 200 in the present disclosure may also include multiple processors, and thus operations of a method that are performed by one processor as described in the present disclosure may also be jointly or separately performed by the multiple processors.

The storage 220 may store data/information obtained from the PET scanner 110, the terminal device 130, the storage device 150, or any other component of the PET system 100. In some embodiments, the storage 220 may include a mass storage device, a removable storage device, a volatile read-and-write memory, a read-only memory (ROM), or the like, or any combination thereof. In some embodiments, the storage 220 may store one or more programs and/or instructions to perform exemplary methods described in the present disclosure.

The I/O 230 may input or output signals, data, or information. In some embodiments, the I/O 230 may enable user interaction with the processing device 140. In some embodiments, the I/O 230 may include an input device and an output device.

The communication port 240 may be connected to a network (e.g., the network 120) to facilitate data communications. The communication port 240 may establish connections between the processing device 140 and the PET scanner 110, the terminal device 130, or the storage device 150. The connection may be a wired connection, a wireless connection, or combination of both that enables data transmission and reception.

It should be noted that the above description of the computing device 200 is merely provided for the purposes of illustration, and not intended to limit the scope of the present disclosure. For persons having ordinary skills in the art, multiple variations and modifications may be made under the teachings of the present disclosure.

FIG. 3 is a block diagram illustrating exemplary processing device according to some embodiments of the present disclosure. As shown in FIG. 3, the processing device 140 may include an obtaining module 310, a determination module 320, and a generation module 330. As described in FIG. 1, the PET system 100 in the present disclosure may also include multiple processing devices, and the obtaining module 310, the determination module 320, and the generation module 330 may be components of different processing devices.

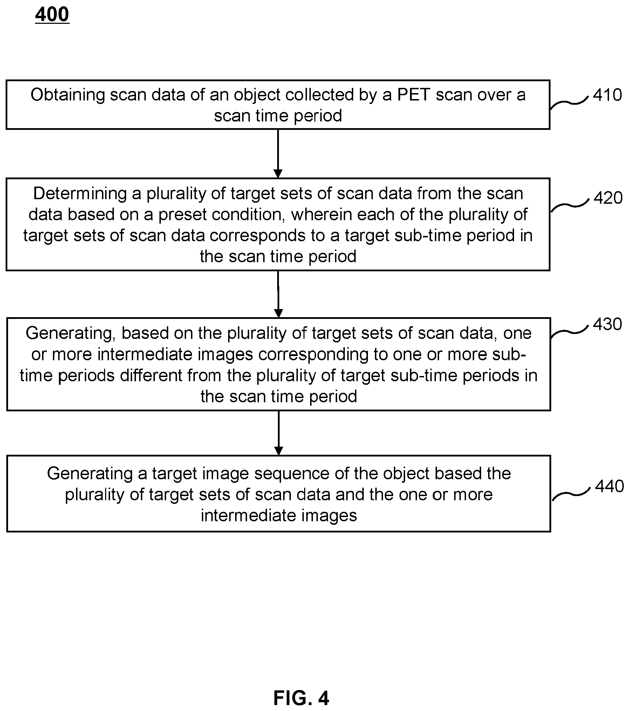

The obtaining module 310 may be configured to obtain data and/or information relating to the PET system 100. For example, the obtaining module 310 may obtain scan data of an object collected by a PET scan over a scan time period. More descriptions regarding the obtaining of the scan data may be found elsewhere in the present disclosure. See, e.g., operation 410 in FIG. 4, and relevant descriptions thereof.

The determination module 320 may be configured to determine a plurality of target sets of scan data from the scan data based on a preset condition. Each of the plurality of target sets of scan data may correspond to a target sub-time period in the scan time period. More descriptions regarding the determinations of the plurality of target sets of scan data may be found elsewhere in the present disclosure. See, e.g., operations 420 in FIG. 4, and relevant descriptions thereof.

The generation module 330 may be configured to generate one or more intermediate images corresponding to one or more sub-time periods different from the plurality of target sub-time periods in the scan time period based on the plurality of target sets of scan data. The generation module 330 may be also configured to generate a target image sequence of the object based on the target sets of scan data and the one or more intermediate images. More descriptions regarding the generations of the one or more intermediate images and the target image sequence may be found elsewhere in the present disclosure. See, e.g., operations 430 and 440 in FIG. 4, and relevant descriptions thereof.

It should be noted that the above description is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, any one of the modules may be divided into two or more units. For instance, the obtaining module 310 may be divided into two units configured to acquire different data. In some embodiments, the processing device 140 may include one or more additional modules, such as a storage module (not shown) for storing data.

FIG. 4 is a flowchart illustrating an exemplary process for generating a target image sequence of an object during a dynamic PET imaging according to some embodiments of the present disclosure. In some embodiments, process 400 may be executed by the PET system 100. For example, the process 400 may be implemented as a set of instructions (e.g., an application) stored in a storage device (e.g., the storage device 150, the storage 220). In some embodiments, the processing device 140 (e.g., the processor 210 of the computing device 200 and/or one or more modules illustrated in FIG. 3) may execute the set of instructions and may accordingly be directed to perform the process 400. The operations of the illustrated process presented below are intended to be illustrative. In some embodiments, the process 400 may be accomplished with one or more additional operations not described and/or without one or more of the operations discussed. Additionally, the order of the operations of process 400 illustrated in FIG. 4 and described below is not intended to be limiting.

In 410, the processing device 140 (e.g., the obtaining module 310, the processor 210) may obtain scan data of an object (e.g., a patient) collected by a PET scan over a scan time period.

In some embodiments, the processing device 140 may obtain the scan data collected by a dynamic PET scan (also referred to as a "dynamic PET imaging") performed by the PET scanner 110. In some embodiments, the scan time period may be a predetermined period (e.g., 1-5 minutes, 5-10 minutes, 5-15 minutes, 10-20 minutes) during the dynamic PET scan. In some embodiments, a form of the scan data may include a listmode form, a sonogram form, a histo-image form, a histo-projection form, etc.

In some embodiments, the processing device 140 may obtain the scan data from one or more components (e.g., the PET scanner 110, the storage device 150) of the PET system 100 or an external source via a network (e.g., the network 120).

In 420, the processing device 140 (e.g., the determination module 320, the processor 210) may determine a plurality of target sets of scan data from the scan data based on a preset condition, wherein each of the plurality of target sets of scan data corresponds to a target sub-time period in the scan time period.

The preset condition may indicate a condition that the plurality of target sets of scan data need to satisfy. For example, the preset condition may be that a difference between two adjacent target sets of scan data among the plurality of target sets of scan data is relatively large, for example, larger than a difference threshold which may be a default setting of the PET system 100 or may be adjustable under different situations.

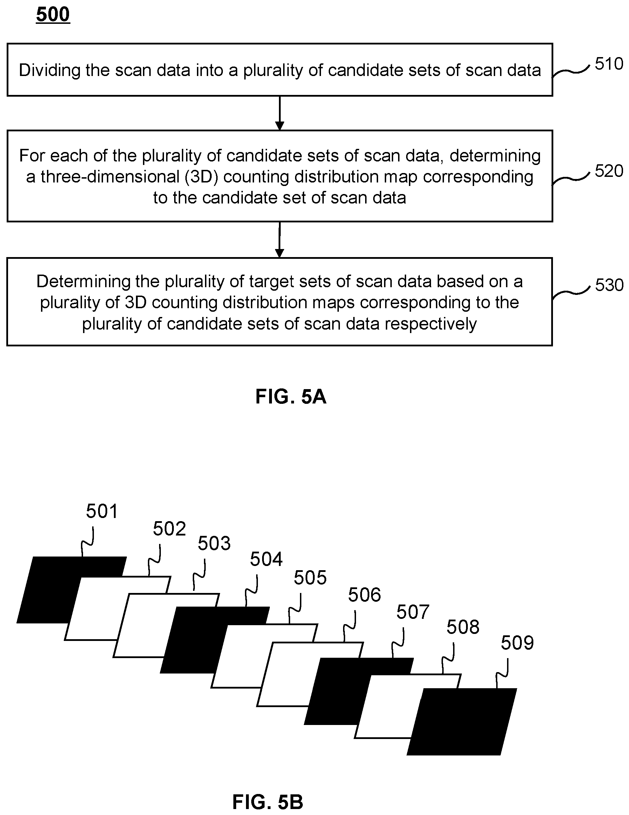

In some embodiments, the processing device 140 may divide the scan data into a plurality of candidate sets of scan data. For each of the plurality of candidate sets of scan data, the processing device 140 may determine a three-dimensional (3D) counting distribution map corresponding to the candidate set of scan data. The 3D counting distribution map may include at least one pixel each of which corresponds to a pixel value indicating a count of coincidence events associated with the pixel. Further, the processing device 140 may determine the plurality of target sets of scan data based on a plurality of 3D counting distribution maps corresponding to the plurality of candidate sets of scan data respectively. More descriptions regarding the determination of the plurality of target sets of scan data based on the plurality of 3D counting distribution maps may be found elsewhere in the present disclosure, for example, FIG. 5A and FIG. 5B, and the descriptions thereof.

In some embodiments, the scan time period may include multiple sub-time periods (corresponding to multiple sets of scan data respectively). Accordingly, the processing device 140 may determine a plurality of target sub-time periods among the multiple sub-time periods in the scan time period, and designate scan data collected in each of the plurality of target sub-time periods as a target set of scan data. In some embodiments, durations of the plurality of target sub-time periods corresponding to the plurality of target sets of scan data respectively may be the same. Alternatively or optionally, the durations of the plurality of target sub-time periods corresponding to the plurality of target sets of scan data respectively may be different.

In some embodiments, the processing device 140 may determine a first sub-time period and/or a last sub-time period as target sub-time period(s), and determine corresponding set(s) of scan data as target set(s) of scan data. In some embodiments, the processing device 140 may select (e.g., randomly select) some sub-time periods between the first sub-time period and the last sub-time period as target sub-time periods, and determine corresponding sets of scan data as target sets of scan data.

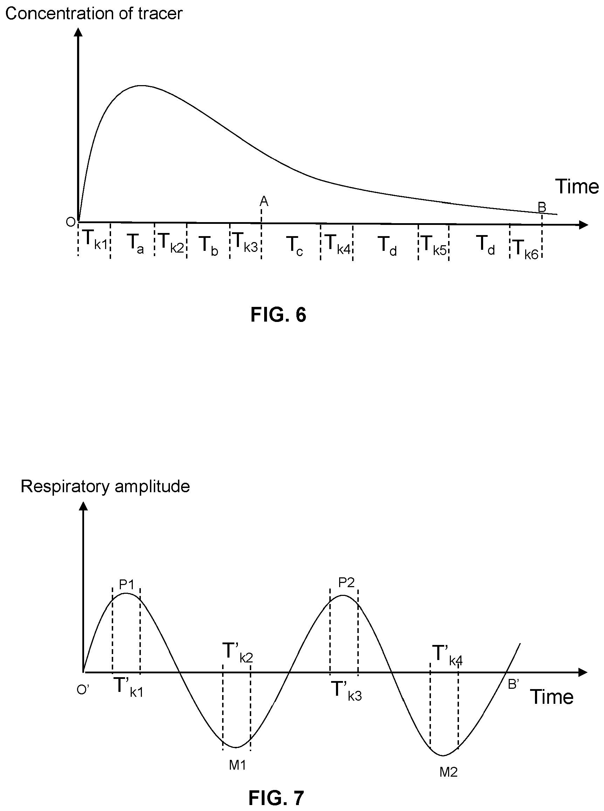

In some embodiments, the processing device 140 may obtain a time-activity curve (TAC) associated with the tracer for the PET scan and determine the plurality of target sub-time periods (and the corresponding plurality of target sets of scan data) based on the TAC. The TAC may indicate changes in a concentration of the tracer in the object over time. In some embodiments, the TAC may be a TAC of the object generated based on the scan data of the object. In some embodiments, the TAC may be a population-based TAC determined based on statistics of historical data. In some embodiments, the processing device 140 may obtain the TAC from the storage device 150 or an external resource. Merely by way of example, FIG. 6 is a schematic diagram illustrating an exemplary TAC according to some embodiments of the present disclosure. As shown in FIG. 6, a horizontal axis of the TAC represents time, and a vertical axis of the TAC represents a concentration of the tracer.

In some embodiments, the processing device 140 may define a target duration and determine the plurality of target sub-time periods based on the target duration and a concentration change (which may be defined by a slope of the TAC) of the tracer. For example, if the concentration change of the tracer is relatively large over a time period (i.e., the slope of the TAC is relatively large over the time period), time intervals between adjacent target sub-time periods within the time period may be relatively small; if the concentration change of the tracer is relatively small over a time period (i.e., the slope of the TAC is relatively small over the time period), the time intervals between adjacent target sub-time periods within the time period may be relatively large, wherein all the plurality of target sub-time periods correspond to the same target duration. Specifically, for example, as shown in FIG. 6, $T_{k1}$, $T_{k2}$, $T_{k3}$, $T_{k4}$, $T_{k5}$, and $T_{k6}$ refer to target sub-time periods. It can be seen that the concentration change of the tracer is relatively large over a time period OA, that is, the slope of the TAC is relatively large over the time period OA, accordingly, the time intervals $T_a$ and $T_b$ between adjacent target sub-time periods $T_{k1}$, $T_{k2}$, and $T_{k3}$ within the time period OA are relatively small; whereas, the concentration change of the tracer is relatively small over a time period AB, that is, the slope of the TAC is relatively small over the time period OB, accordingly, the time intervals $T_c$ and $T_d$ between adjacent target sub-time periods $T_{k3}$, $T_{k4}$, $T_{k5}$, and $T_{k6}$ are relatively large.

In some embodiments, the processing device 140 may obtain a vital signal of the object corresponding to the scan data and determine the plurality of target sub-time periods (and the corresponding plurality of target sets of scan data) based on the vital signal. In some embodiments, the vital signal refers to a physiological signal generated by a physiological motion of the object, such as a cardiac motion, a respiratory motion, etc. Exemplary vital signals may include a heartbeat signal, a breathing signal, or the like, or any combination thereof. In some embodiments, the vital signal may be collected via a vital signal monitor and/or stored in a storage device (e.g., the storage device 150). The processing device 140 may obtain the vital signal from the vital signal monitor and/or the storage device. Merely by way of example, FIG. 7 is a schematic diagram illustrating an exemplary respiratory curve according to some embodiments of the present disclosure. As shown in FIG. 7, a horizontal axis of the respiratory curve represents time, and a vertical axis of the respiratory curve represents a respiratory amplitude.

In some embodiments, the processing device 140 may determine define a target duration and determine the plurality of target sub-time periods based on the target duration and peaks and/or valleys of the respiratory curve. For example, the processing device 140 may determine a time period $T'_{k1}$ (duration of which is the target duration) including a peak P1, a time period $T'_{k2}$ (duration of which is the target duration) including a peak P2, a time period $T'_{k3}$ (duration of which is the target duration) including a valley M1, a time period $T'_{k4}$ (duration of which is the target duration) including a valley M2 as target sub-time periods.

In some embodiments, the processing device 140 may define a target duration (e.g., 0.1 seconds, 0.5 seconds, 1 second) and a target time interval (e.g., 3 seconds, 5 seconds, 8 seconds) between adjacent target sub-time periods, and determine the plurality of target sub-time periods based on the target duration and the target time interval. Further, the processing device 140 may determine corresponding sets of scan data within the plurality of target sub-time periods as the plurality of target sets of scan data.

In 430, the processing device 140 (e.g., the generation module 330, the processor 210) may generate, based on the plurality of target sets of scan data, one or more intermediate images corresponding to one or more sub-time periods different from the plurality of target sub-time periods in the scan time period.

For brief, a sub-time period corresponding to an intermediate image may be referred to as an intermediate sub-time period. In some embodiments, the one or more intermediate sub-time periods may include one or more sub-time periods other than the plurality of target sub-time periods. In some embodiments, the one or more intermediate sub-time periods and the plurality of target sub-time periods may be partially overlapped.

In some embodiments, the processing device 140 may generate a plurality of target images corresponding to the plurality of target sets of scan data respectively, and generate the one or more intermediate images based on the plurality of target images.

In some embodiments, for each of the plurality of target sets of scan data, the processing device 140 may determine a preliminary image based on the target set of scan data using an image reconstruction technique. Exemplary image reconstruction techniques may include filtered back projection (FBP), an algebraic reconstruction technology (ART), a statistical reconstruction (SR) algorithm, or the like, or any combination thereof. It should be understood by those skilled in the art that the image reconstruction technique may be varied. All such variations are within the protection scope of the present disclosure.

In some embodiments, the processing device 140 may generate the plurality of preliminary images using an image reconstruction model. The image reconstruction model may be a trained model (e.g., a machine learning model) for reconstructing PET images based on PET scan data. In some embodiments, the image reconstruction model may be trained based on a plurality of training samples each of which includes sample scan data of a sample object collected by a PET scan and a reference image of the sample object, wherein the reference image can be used as a ground truth (also referred to as a label) for model training.

In some embodiments, the processing device 140 may designate the plurality of preliminary images corresponding to the plurality of target sets of scan data as the plurality of target images corresponding to the plurality of target sets of scan data.

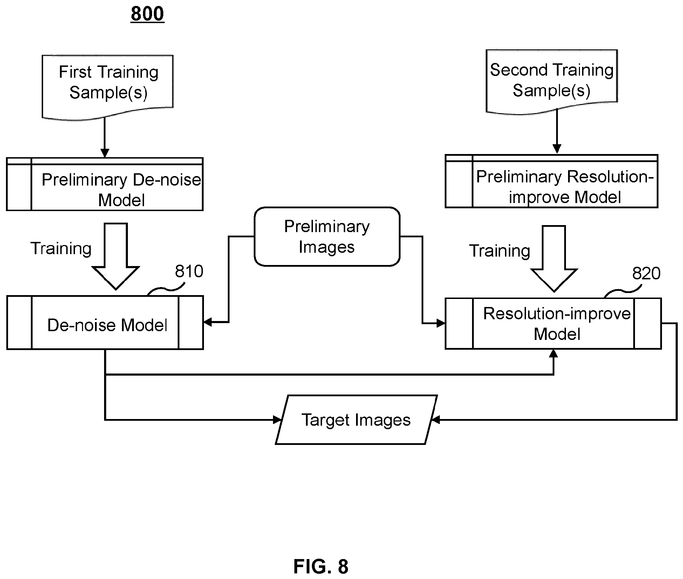

In some embodiments, the processing device 140 may determine the plurality of target images by performing a de-noise operation on the plurality of preliminary images. In some embodiments, the de-noise operation may be performed according to a de-noise algorithm. Exemplary de-noise algorithms may include a mean filtering algorithm, an adaptive Wiener filtering algorithm, a median filtering algorithm, a wavelet denoising, etc. In some embodiments, the de-noise operation may be performed according to a de-noise model (e.g., a de-noise model 810 illustrated in FIG. 8). In some embodiments, the de-noise model may be a trained model (e.g., a machine learning model) for reducing noise in an image. In some embodiments, the de-noise model may include a deep learning model, such as a deep neural network (DNN) model, a convolutional neural network (CNN) model, a recurrent neural network (RNN) model, a feature pyramid network (FPN) model, etc. Exemplary CNN models may include a V-Net model, a U-Net model, a Link-Net model, or the like, or any combination thereof.

In some embodiments, the de-noise model may be trained based on a plurality of training samples. Merely by way of example, FIG. 8 is a schematic diagram illustrating an exemplary process for generating a plurality of target images according to some embodiments of the present disclosure. As illustrated in FIG. 8, the de-noise model may be determined by training a preliminary de-noise model based on one or more first training samples. In some embodiments, each first training sample may include a sample image of a sample object and a reference image of the sample object, wherein the reference image has relatively low noise and can be used as a ground truth (also referred to as a label) for model training. In some embodiments, the reference image may be defined by a user or may be automatically determined by a training device. In some embodiments, the sample object may be the same as or similar to the object described in elsewhere in the present disclosure. In some embodiments, the reference image may be generated based on sample data of the sample object collected by a sample PET scan using any image reconstruction technique described in connection with operation 430. The sample PET scan may be performed for a relatively long time to obtain enough sample scan data. In some embodiments, a uniform down-sampling operation may be performed on at least a portion of the sample data and the sample image may be generated based on the down-sampled sample data using any image reconstruction technique described in connection with operation 430.

In some embodiments, the preliminary de-noise model may be trained iteratively until a termination condition is satisfied. In some embodiments, the termination condition may relate to a value of a loss function. For example, the termination condition may be deemed satisfied if the value of the loss function is minimal or smaller than a predetermined threshold. As another example, the termination condition may be deemed satisfied if the value of the loss function converges. In some embodiments, "convergence" may refer to that the variation of the values of the loss function in two or more consecutive iterations is equal to or smaller than a predetermined threshold. In some embodiments, "convergence" may refer to that a difference between the value of the loss function and a target value is equal to or smaller than a predetermined threshold. In some embodiments, the termination condition may be deemed satisfied when a specified count of iterations have been performed in the training process.

In some embodiments, the processing device 140 may determine the plurality of target images by performing a resolution-improve operation on the plurality of preliminary images. In some embodiments, the resolution-improve operation may be performed according to a resolution-improve algorithm, such as an interpolation method. In some embodiments, the resolution-improve operation may be performed according to a resolution-improve model (e.g., a resolution-improve model 820 illustrated in FIG. 8). In some embodiments, the resolution-improve model may be a trained model (e.g., a machine learning model) for improving resolution of an image. In some embodiments, a type of the resolution-improve model may be the same as that of the de-noise model.

In some embodiments, the resolution-improve model may be trained based on a plurality of training samples. Merely by way of example, as illustrated in FIG. 8, the resolution-improve model may be determined by training a preliminary resolution-improve model based on one or more second training samples. In some embodiments, each second training sample may include a sample image of a sample object and a reference image of the sample object, wherein the reference image has a relatively high resolution and can be used as a ground truth (also referred to as a label) for model training. In some embodiments, the sample image may correspond to a relatively large pixel size (e.g., a pixel size of 4 mm x 4 mm), and the reference image may correspond to a relatively small pixel size (e.g., a pixel size of 2 mm x 2 mm). In some embodiments, the reference image may be defined by a user or may be automatically determined by a training device. In some embodiments, the sample object may be the same as or similar to the object described in elsewhere in the present disclosure. In some embodiments, the sample image may be generated based on sample data of the sample object collected by a sample PET scan using any image reconstruction technique described in connection with operation 430. In some embodiments, the sample PET scan may be performed for a relatively long time to obtain enough sample scan data. In some embodiments, the preliminary resolution-improve model may be trained iteratively until a termination condition is satisfied. More descriptions may be found above the descriptions of which are not repeated here.

In some embodiments, the processing device 140 may obtain the de-noise model and/or the resolution-improve model from one or more components (e.g., the storage device 150, the storage 210) of the PET system 100 or an external source via a network (e.g., the network 120). For example, the de-noise model and/or the resolution-improve model may be previously trained by a computing device (e.g., the processing device 140 or other processing devices), and stored in a storage device (e.g., the storage device 150, the storage 210) of the PET system 100. The processing device 140 may access the storage device and retrieve the de-noise model and/or the resolution-improve model.

In some embodiments, the processing device 140 may determine the plurality of target images by performing both the de-noise operation and the resolution-improve operation on the plurality of preliminary images. For example, the processing device 140 may determine the plurality of target images by performing the de-noise operation on the plurality of preliminary images using the de-noise model and performing the resolution-improve operation on the plurality of preliminary images using the resolution-improve model. Merely by way of example, as illustrated in FIG. 8, the preliminary images may be input into the de-noise model, the outputs of the de-noise model may be input into the resolution-improve operation, and the resolution-improve model may output the target images corresponding to the preliminary images. In some embodiments, the de-noise model and the resolution-improve model may be integrated into a single model.

According to some embodiments of the present disclosure, the plurality of target images may be obtained by performing the de-noise operation and/or the resolution-improve operation on the plurality of preliminary images, which can improve the image quality of the target images.

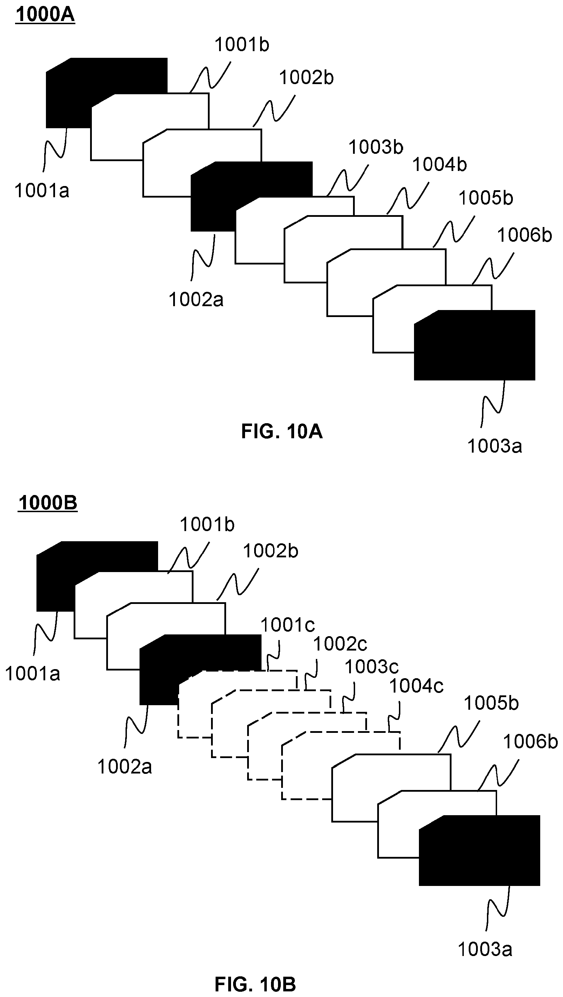

In some embodiments, the processing device 140 may obtain one or more pairs of target images among the plurality of target images. In some embodiments, a pair of target images may be any two target images among the plurality of target images. For example, as illustrated in FIG. 10A, a pair of target images may be two adjacent target images (e.g., 1001a and 1002a, 1002a and 1003a) among the plurality of the target images. In some embodiments, the one or more pairs of target images may be determined manually by a user (e.g., a doctor, an imaging specialist, a technician). In some embodiments, the one or more pairs of target images may be determined by the processing device 140 automatically.

Further, for each pair of the one or more pairs of target images among the plurality of target images, the processing device 140 may generate one or more intermediate images between the pair of target images. For example, as illustrated in FIG. 10A, for the pair of target images 1001a and 1002a, the processing device 140 may generate intermediate images 1001b and 1002b corresponding to the pair of target images 1001a and 1002a; for the pair of target images 1002a and 1003a, the processing device 140 may generate intermediate images 1003b-1006b corresponding to the pair of target images 1002a and 1003a. In some embodiments, durations of intermediate sub-time periods corresponding to multiple intermediate images between the pair of target images may be the same or different. In some embodiments, the durations of the one or more intermediate sub-time periods corresponding to the one or more intermediate images between the pair of target images may be the same as or different from the duration of the target sub-time periods corresponding to the pair of target images (it is assumed that the pair of target images correspond to a same duration).

In some embodiments, for each pair of the one or more pairs of target images, the processing device 140 may determine the one or more intermediate images based on a motion field between the pair of target images.

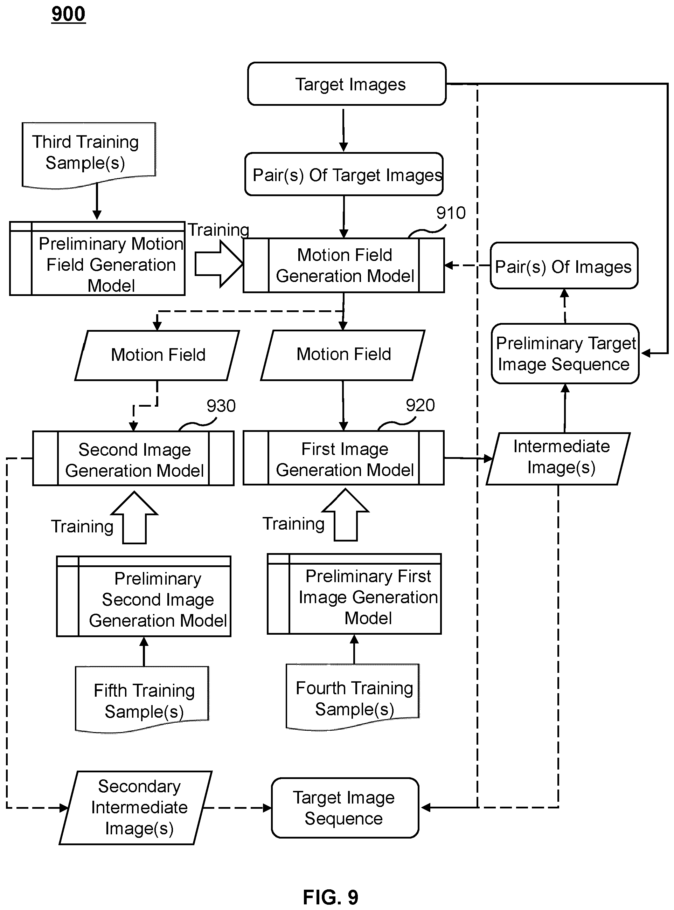

In some embodiments, for each pair of the one or more pairs of target images, the processing device 140 may determine a motion field between the pair of target images using a motion field generation model (e.g., a motion field generation model 910 illustrated in FIG.9) and generate one or more intermediate images corresponding to the pair of target images based on the motion field using an image generation model (e.g., a first image generation model 920 illustrated in FIG. 9).

In some embodiments, the motion field generation model may be a trained model (e.g., a machine learning model) for determining a motion field between two images. In some embodiments, the motion field generation model may include a deep learning model, such as a deep neural network (DNN) model, a convolutional neural network (CNN) model, a recurrent neural network (RNN) model, a feature pyramid network (FPN) model, etc. Exemplary CNN models may include a V-Net model, a U-Net model, a Link-Net model, or the like, or any combination thereof.

In some embodiments, the motion field generation model may be trained based on a plurality of training samples. Merely by way of example, as illustrated in FIG. 9, the motion field generation model may be determined by training a preliminary motion field generation model based on one or more third training samples. In some embodiments, each third training sample may include a pair of sample images of a sample object and a reference motion field between the pair of sample images, wherein the reference motion field can be used as a ground truth (also referred to as a label) for model training. In some embodiments, the reference motion field may be defined by a user or may be automatically determined by a training device. In some embodiments, the sample object may be the same as or similar to the object described in elsewhere in the present disclosure. In some embodiments, the pair of sample images may be generated based on sample data of the sample object collected by a sample PET scan using any image reconstruction technique described in connection with operation 430. In some embodiments, the sample PET scan may be performed for a relatively long time to obtain enough sample scan data. In some embodiments, the preliminary motion field generation model may be trained iteratively until a termination condition is satisfied. More descriptions may be found above the descriptions of which are not repeated here.

In some embodiments, the image generation model may be a trained model (e.g., a machine learning model) for generating one or more intermediate images between two images. In some embodiments, the image generation model may include may include a generative adversarial network (GAN) model, a diffusion model, etc.

In some embodiments, the image generation model may be trained based on a plurality of training samples. Merely by way of example, as illustrated in FIG. 9, the first image generation model may be determined by training a preliminary first image generation model based on one or more fourth training samples. In some embodiments, each fourth training sample may include a pair of sample images of a sample object, a sample motion field between the pair of sample images, and one or more reference intermediate images between the pair of sample images, wherein the one or more reference intermediate images can be used as a ground truth (also referred to as a label) for model training. In some embodiments, the one or more reference intermediate images may be defined by a user or may be automatically determined by a training device.

In some embodiments, the sample object may be the same as or similar to the object described in elsewhere in the present disclosure. In some embodiments, the pair of sample images may be generated based on sample data of the sample object collected by a sample dynamic PET scan using any image reconstruction technique described in connection with operation 430. In some embodiments, the sample dynamic PET scan may be performed for a relatively long time to obtain enough sample scan data. In some embodiments, a plurality of reconstruction images corresponding to a plurality of sample sub-time periods (durations of which may be the same or different) of the sample dynamic PET scan may be generated and a pair of reconstruction images among the plurality of reconstruction images may be designated as a pair of the sample images in the fourth training sample. In some embodiments, a difference between the pair of sample images may be relatively large (e.g., greater than a difference threshold). In some embodiments, one or more reconstruction images between the pair of sample images may be designated as the one or more reference intermediate images. In some embodiments, the durations corresponding to the one or more reference intermediate images may be smaller than or equal to the durations corresponding to the pair of sample images.

In some embodiments, the first preliminary image generation model may be trained iteratively until a termination condition is satisfied. More descriptions may be found above the descriptions of which are not repeated here.

In some embodiments, the processing device 140 may obtain the motion field generation model and/or the image generation model from one or more components (e.g., the storage device 150, the storage 210) of the PET system 100 or an external source via a network (e.g., the network 120). For example, the motion field generation model and/or the image generation model may be previously trained by a computing device (e.g., the processing device 140 or other processing devices), and stored in a storage device (e.g., the storage device 150, the storage 210) of the PET system 100. The processing device 140 may access the storage device and retrieve the motion field generation model and/or the image generation model.

In some embodiments, at least two of the image reconstruction model, the de-noise model, the resolution-improve model, the motion field generation model, or the image generation model may be integrated into a single model. For example, the motion field generation model and the image generation model may be integrated into a single model. As still another example, the image reconstruction model and the motion field generation model may be integrated into a single model. As another example, the image reconstruction model, the motion field generation model, and the image generation model may be integrated into a single model. As still another example, the image reconstruction model, the de-noise model, the resolution-improve model, the motion field generation model, and the image generation mode may be integrated into a single model.

In some embodiments, the motion field generation model may also be configured to generate the plurality of target images based on the plurality of target sets of the scan data. The processing device 140 may determine the motion field by directly inputting the plurality of target sets of scan data into the motion field generation model.

In some embodiments, the motion field generation model may also be configured to generate the one or more intermediate images based on the plurality of target sets of the scan data or the plurality of target images. For example, the processing device 140 may determine the one or more intermediate images by inputting the plurality of target sets of scan data into the motion field generation model. As another example, the processing device 140 may determine the one or more intermediate images by inputting the plurality of target images into the motion field generation model.

In 440, the processing device 140 (e.g., the generation module 330, the processor 210) may generate a target image sequence of the object based on the plurality of target sets of scan data and the one or more intermediate images.

In some embodiments, the processing device 140 may arrange the plurality of target images and the one or more intermediate images in chronological order of the target sub-time periods and the intermediate sub-time periods to form the target image sequence. For example, FIG. 10A is a schematic diagram illustrating an exemplary target image sequence according to some embodiments of the present disclosure. As shown in FIG. 10A, the target images 1001a, 1002a, and 1003a and the intermediate images 1001b, 1002b, 1003b, 1004b, 1005b, and 1006b are arranged in order (e.g., 1001a, 1001b, 1002b, 1002a, 1003b, 1004b, 1005b, 1006b, and 1003a) to form the target image sequence 1000A.

In some embodiments, the processing device 140 may obtain one or more pairs of images among the arranged plurality of target images and the one or more intermediate images (also can be referred to as a "preliminary target image sequence"). Similar to above, a pair of images may be any two images (e.g., 1002a and 1005b shown in FIG. 10B) among the preliminary target image sequence. In some embodiments, a difference between each pair of the one or more pairs of images may be larger than a preset difference threshold, which may be a default setting of the PET system 100 or may be adjustable under different situations. In some embodiments, the one or more pairs of images may be determined manually by a user (e.g., a doctor, an imaging specialist, a technician). In some embodiments, the one or more pairs of images may be determined by the processing device 140 automatically.

Further, for each pair of the one or more pairs of images among the preliminary target image sequence, the processing device 140 may generate one or more secondary intermediate images between the pair of images. For example, as illustrated in FIG. 10B, for the pair of images 1002a and 1005b, the processing device 140 may generate secondary intermediate images 1001c, 1002c, 1003c, and 1004c corresponding to the pair of images 1002a and 1005b. In some embodiments, durations corresponding to the one or more secondary intermediate images may be smaller than or equal to durations corresponding to the pair of images.

In some embodiments, for each pair of the one or more pairs of images, the processing device 140 may determine a motion field (also can be referred to as a "secondary motion field") between the pair of images using a motion field generation model (e.g., the motion field generation model 910 illustrated in FIG. 9) and generate one or more secondary intermediate images corresponding to the pair of images based on the motion field using a second image generation model (e.g., a second image generation model 930 illustrated in FIG. 9). In some embodiments, the second image generation model may be a trained model (e.g., a machine learning model) for generating one or more intermediate images between two images. In some embodiments, the second image generation model may be the same as or similar to the image generation model (also can be referred to as a "first image generation model").

In some embodiments, the second image generation model may be trained based on a plurality of training samples. Merely by way of example, as illustrated in FIG. 9, the second image generation model may be determined by training a preliminary second image generation model based on one or more fifth training samples. In some embodiments, each fifth training sample may include a pair of sample images of a sample object, a sample motion field between the pair of sample images, and one or more reference intermediate images between the pair of sample images, wherein the one or more reference intermediate images can be used as a ground truth (also referred to as a label) for model training. In some embodiments, the one or more reference intermediate images may be defined by a user or may be automatically determined by a training device.

In some embodiments, the sample object may be the same as or similar to the object described in elsewhere in the present disclosure. In some embodiments, the pair of sample images may be generated based on sample data of the sample object collected by a sample dynamic PET scan using any image reconstruction technique described in connection with operation 430. In some embodiments, the sample dynamic PET scan may be performed for a relatively long time to obtain enough sample scan data. In some embodiments, a plurality of reconstruction images corresponding to a plurality of sample sub-time periods (durations of which may be the same or different) of the sample dynamic PET scan may be generated and a pair of reconstruction images among the plurality of reconstruction images may be designated as a pair of the sample images in the fifth training sample. In some embodiments, a difference between the pair of sample images may be relatively large (e.g., greater than a difference threshold). In some embodiments, one or more reconstruction images between the pair of sample images may be designated as the one or more reference intermediate images. In some embodiments, durations corresponding to the one or more reference intermediate images in the fifth training sample may be smaller than the durations corresponding to the one or more reference intermediate images in the fourth training sample.

In some embodiments, the second image generation model may be integrated into the image generation model.

Furthermore, the processing device 140 may generate the target image sequence of the object based on the plurality of target images, the one or more intermediate images, and the one or more secondary intermediate images corresponding to the one or more pairs of images. In some embodiments, similarly, the processing device 140 may arrange the plurality of target images, the one or more intermediate images, and the one or more secondary intermediate images corresponding to the one or more pairs of images in chronological order of the target sub-time periods and the intermediate sub-time periods to form the target image sequence. For example, FIG. 10B is a schematic diagram illustrating an exemplary target image sequence according to some embodiments of the present disclosure. As shown in FIG. 10B, the target images 1001a, 1002a, and 1003a, the intermediate images 1001b, 1002b, 1005b, and 1006b, and the secondary intermediate images 1001c, 1002c, 1003c, and 1004c are arranged in order (e.g., 1001a, 1001b, 1002b, 1002a, 1001c, 1002c, 1003c, 1004c, 1005b, 1006b, and 1003a) to form the target image sequence 1000B.

According to some embodiments of the present disclosure, intermediate image(s) may be generated (e.g., based on a motion field between a pair of target images) without image reconstruction, accordingly, an image reconstruction time can be reduced. Further, the intermediate image(s) may be generated according to a motion field generation model and an image generation model, and/or before the generation of the target images, a de-noise operation may be performed using a de-noise model and/or a resolution-improve operation may be performed using a resolution-improve model. Accordingly, the imaging efficiency and the image quality can be improved.

In some embodiments, if a trace for the PET scan includes a plurality of types of tracers, the processing device 140 may determine scan data corresponding to each type of tracer and accordingly determine a target image sequence corresponding to each type of tracer by performing the above operations 410-450.

It should be noted that the above description regarding the process 400 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, the process 400 may be accomplished with one or more additional operations not described and/or without one or more of the operations discussed above.

FIG. 5A is a flowchart illustrating an exemplary process for determining a plurality of target sets of scan data according to some embodiments of the present disclosure. In some embodiments, process 500 may be executed by the PET system 100. For example, the process 500 may be implemented as a set of instructions (e.g., an application) stored in a storage device (e.g., the storage device 150, the storage 220). In some embodiments, the processing device 140 (e.g., the processor 210 of the computing device 200 and/or one or more modules illustrated in FIG. 3) may execute the set of instructions and may accordingly be directed to perform the process 500. The operations of the illustrated process presented below are intended to be illustrative. In some embodiments, the process 500 may be accomplished with one or more additional operations not described and/or without one or more of the operations discussed. Additionally, the order of the operations of process 500 illustrated in FIG. 5A and described below is not intended to be limiting. In some embodiments, one or more operations of the process 500 may be performed to achieve at least part of operation 420 as described in connection with FIG. 4.

In 510, the processing device 140 (e.g., the determination module 320, the processor 210) may divide the scan data into a plurality of candidate sets of scan data.

In some embodiments, as described above, the scan data may be collected by a dynamic PET imaging over a dynamic scan time period. Accordingly, the processing device 140 may divide the dynamic scan time period into a plurality of candidate time periods. Further, the processing device 140 may divide the scan data into the plurality of candidate sets of scan data according to the plurality of candidate time periods. Specifically, the processing device 140 may designate scan data collected in each of the plurality of candidate time periods as one set of the plurality of candidate sets of scan data.

In 520, for each of the plurality of candidate sets of scan data, the processing device 140 (e.g., the determination module 320, the processor 210) may determine a three-dimensional (3D) counting distribution map corresponding to the candidate set of scan data.

In some embodiments, the 3D counting distribution map may include at least one pixel each of which corresponds to a pixel value indicating a count of coincidence events associated with the pixel.

In some embodiments, for each coincidence event in the candidate set of scan data, the processing device 140 may determine an annihilation position of annihilation reaction corresponding to the coincidence event. In some embodiments, the annihilation position may be represented by a maximum likelihood coordinate corresponding to the coincidence event. Merely by way of example, the processing device 140 may determine the maximum likelihood coordinate corresponding to the coincidence event according to according to Equation (1) shown below:

$$\vec{x}_0 = Tbin \cdot TbinSize \cdot \frac{(\vec{x}_b - \vec{x}_a)}{|\vec{x}_b - \vec{x}_a|} + \frac{\vec{x}_b + \vec{x}_a}{2}, \tag{1}$$

where $\vec{x}_0$ denotes the maximum likelihood coordinate corresponding to the coincidence event, $Tbin$ denotes a serial number of an interval of the TOF on a LOR corresponding to the coincidence event, $TbinSize$ denotes a width of the interval of the TOF, and $\vec{x}_a$ and $\vec{x}_b$ denote coordinates of two detector units detecting the coincidence event.

Further, the processing device 140 may determine the 3D counting distribution map based on annihilation positions corresponding to the coincidence events in the candidate set of scan data. For example, the processing device 140 may obtain an initial 3D counting distribution map including a field of vision of the PET scanner for the PET scan. Each pixel of the initial 3D counting distribution map may correspond to a spatial location and have a pixel value of 0. For each pixel of the initial 3D counting distribution map, the processing device 140 may determine a count of coincidence events corresponding to the pixel based on the annihilation positions corresponding to the coincidence events in the candidate set of scan data. The count of coincidence events corresponding to the pixel refers to a count of annihilation reactions occurring at the spatial location corresponding to the pixel. The processing device 140 may designate the count of coincidence events corresponding to the pixel as a new pixel value of the pixel. Further, the processing device 140 may update the initial 3D counting distribution map using new pixel values of the pixels in the initial 3D counting distribution map to obtain the 3D counting distribution map corresponding to the candidate set of scan data.

In 530, the processing device 140 (e.g., the determination module 320, the processor 210) may determine the plurality of target sets of scan data based on a plurality of 3D counting distribution maps corresponding to the plurality of candidate sets of scan data respectively.

In some embodiments, the plurality of 3D counting distribution maps corresponding to the plurality of candidate sets of scan data may be arranged in chronological order of the plurality of candidate time periods to form a map sequence. The processing device 140 may determine the plurality of target sets of scan data by traversing the map sequence starting from the first 3D counting distribution map in the map sequence. Specifically, the processing device 140 may determine a difference between the latest 3D counting distribution map corresponding to the latest determined target set of scan data and each of the 3D counting distribution maps after the latest 3D counting distribution map in sequence until the difference between the latest 3D counting distribution map and one of the 3D counting distribution maps after the latest 3D counting distribution map is larger than or equal to a preset threshold, the processing device 140 may designate a candidate set of scan data corresponding to the one of the 3D counting distribution maps as a target set of scan data. As used herein, each time a target set of scan data is determined, the target set of scan data is considered as the latest determined target set of scan data, and the 3D counting distribution map corresponding to the latest determined target set of scan data is considered as the latest 3D counting distribution map. For example, the processing device 140 may designate a candidate set of scan data corresponding to the first 3D counting distribution map as a target set of scan data. The processing device 140 may determine a difference between the first 3D counting distribution map and a next adjacent 3D counting distribution map. In response to determining the difference is larger than or equal to a preset threshold, the processing device 140 designate the candidate set of scan data corresponding to the next adjacent 3D counting distribution map as a target set of scan data, and then continue to determine the difference between the next adjacent 3D counting distribution map and a next adjacent 3D counting distribution map of the next adjacent 3D counting distribution map. In response to determining the difference is smaller than the preset threshold, the processing device 140 may determine a difference between the first 3D counting distribution map and a next adjacent 3D counting distribution map of the next adjacent 3D counting distribution map.

For example, FIG. 5B is a schematic diagram illustrating exemplary 3D counting distribution maps according to some embodiments of the present disclosure. As shown in FIG. 5B, a plurality of 3D counting distribution maps 501-509 corresponding to a plurality of candidate sets of scan data respectively are arranged in chronological order of the candidate time periods. The processing device 140 may determine a difference between a first 3D counting distribution map 501 and a second 3D counting distribution map 502. In response to determining that the difference is smaller than the preset threshold, the processing device 140 may determine a difference between the first 3D counting distribution map 501 and a third 3D counting distribution map 503. In response to determining that the difference is smaller than the preset threshold, the processing device 140 may further determine a difference between the first 3D counting distribution map 501 and a fourth 3D counting distribution map 504. In response to determining that the difference is larger than the preset threshold, the processing device 140 may designate the candidate set of scan data corresponding to the fourth 3D counting distribution map 504 as a target set of scan data. Then the processing device 140 may determine a difference between the fourth 3D counting distribution map 504 and a fifth 3D counting distribution map 505, and so on, until all the 3D counting distribution maps are traversed. As shown in FIG. 5B, the finally determined target sets of scan data correspond to 3D counting distribution maps 501, 504, 507, and 509.

It will be apparent to those skilled in the art that various changes and modifications can be made in the present disclosure without departing from the scope of the disclosure. In this manner, the present disclosure may be intended to include such modifications and variations if the modifications and variations of the present disclosure are within the scope of the appended claims.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the scope of the exemplary embodiments of this disclosure.

Moreover, certain terminology has been used to describe embodiments of the present disclosure. For example, the terms "one embodiment," "an embodiment," and "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "module," "unit," "component," "device," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of a carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an subject oriented programming language such as Java, Scala, Smalltalk, Eiffel, JADE, Emerald, C++, C#, VB. NET, Python or the like, conventional procedural programming languages, such as the "C" programming language, Visual Basic, Fortran 2003, Perl, COBOL 2002, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications that are within the scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claim subject matter lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate a certain variation (e.g., $\pm 1\%$, $\pm 5\%$, $\pm 10\%$, or $\pm 20\%$) of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable. In some embodiments, a classification condition used in classification or determination is provided for illustration purposes and modified according to different situations. For example, a classification condition that "a value is greater than the threshold value" may further include or exclude a condition that "the probability value is equal to the threshold value."

## Claims

1. A system (100) for positron emission tomography (PET) imaging, comprising:

   at least one storage device (150) including a set of instructions for medical imaging; and
   at least one processor (140) in communication with the at least one storage device (150), wherein when executing the set of instructions, the at least one processor (140) is configured to direct the system (100) to perform operations including:

   (410) obtaining scan data of an object (118) collected by a PET scan over a scan time period; **characterised by**
   (420) determining a plurality of target sets of scan data from the scan data based on a preset condition, wherein each of the plurality of target sets of scan data corresponds to a target sub-time period in the scan time period;

(430) generating, based on the plurality of target sets of scan data, one or more intermediate images corresponding to one or more sub-time periods different from the plurality of target sub-time periods in the scan time period; and

(440) generating a target image sequence of the object (118) based on the plurality of target sets of scan data and the one or more intermediate images.

2. The system (100) of claim 1, wherein the determining a plurality of target sets of scan data from the scan data based on a preset condition includes:

(510) dividing the scan data into a plurality of candidate sets of scan data;

(520) for each of the plurality of candidate sets of scan data, determining a three-dimensional (3D) counting distribution map corresponding to the candidate set of scan data, wherein the 3D counting distribution map includes at least one pixel each of which corresponds to a pixel value indicating a count of coincidence events associated with the pixel; and

(530) determining the plurality of target sets of scan data based on a plurality of 3D counting distribution maps corresponding to the plurality of candidate sets of scan data respectively.

3. The system (100) of claim 2, wherein the determining the plurality of target sets of scan data based on a plurality of 3D counting distribution maps corresponding to the plurality of candidate sets of scan data respectively includes:

arranging the plurality of 3D counting distribution maps in chronological order to form a map sequence; and determining the plurality of target sets of scan data by traversing the map sequence starting from the first 3D counting distribution map in the map sequence, wherein the traversing the map sequence starting from the first 3D counting distribution map includes:

determining a difference between the latest 3D counting distribution map corresponding to the latest determined target set of scan data and each of the 3D counting distribution maps after the latest 3D counting distribution map in sequence until the difference between the latest 3D counting distribution map and one of the 3D counting distribution maps after the latest 3D counting distribution map is larger than or equal to a preset threshold, and designating a candidate set of scan data corresponding to the one of the 3D counting distribution maps as a target set of scan data.

4. The system (100) of claim 1, wherein the determining a plurality of target sets of scan data from the scan data based on a preset condition includes:

obtaining a time-activity curve associated with a tracer for the PET scan; and determining the plurality of target sets of scan data from the scan data based on the time-activity curve.

5. The system (100) of claim 1, wherein the determining a plurality of target sets of scan data from the scan data based on a preset condition includes:

obtaining a vital signal of the object (118) corresponding to the scan data; determining the plurality of target sets of scan data from the scan data based on the vital signal.

6. The system (100) of any one of claims 1-5, wherein the generating one or more intermediate images correspond to one or more sub-time periods different from the plurality of target sub-time periods in the scan time period includes:

generating a plurality of target images corresponding to the plurality of target sets of scan data respectively; and generating the one or more intermediate images based on the plurality of target images.

7. The system (100) of claim 6, wherein the generating a plurality of target images corresponding to the plurality of target sets of scan data respectively includes:

determining a plurality of preliminary images corresponding to the plurality of target sets of scan data respectively; and

determining the plurality of target images by performing a de-noise operation on the plurality of preliminary images using a de-noise model and/or performing a resolution-improve operation on the plurality of preliminary images using a resolution-improve model.

8. The system (100) of claims 6 or 7, wherein the generating one or more intermediate images correspond to one or more sub-time periods different from the plurality of target sub-time periods in the scan time period includes:
for each pair of one or more pairs of target images among the plurality of target images,

determining a motion field between the pair of target images using a motion field generation model; and generating one or more intermediate images corresponding to the pair of target images based on the motion field using an image generation model.

9. The system (100) of claim 8, wherein the motion field generation model and the image generation model are integrated into a single model.

10. The system (100) of any one of claims 6-9, wherein the generating a target image sequence of the object (118) based on the plurality of target sets of scan data and the one or more intermediate images includes:

for each pair of one or more pairs of images among the plurality of target images and the one or more intermediate images,

determining a secondary motion field between the pair of images using a motion field generation model; and generating one or more secondary intermediate images corresponding to the pair of images based on the secondary motion field using an image generation model; and

generating the target image sequence of the object (118) based on the plurality of target images, the one or more intermediate images, one or more secondary intermediate images.

11. The system (100) of claim 10, wherein a difference between the pair of images is larger than a preset difference threshold.

12. A method for positron emission tomography (PET) imaging, the method being implemented on a computing device (200) having at least one storage device (220) and at least one processor (210), the method comprising:

(410) obtaining scan data of an object (118) collected by a PET scan over a scan time period; **characterised by** (420) determining a plurality of target sets of scan data from the scan data based on a preset condition, wherein each of the plurality of target sets of scan data corresponds to a target sub-time period in the scan time period; (430) generating, based on the plurality of target sets of scan data, one or more intermediate images corresponding to one or more sub-time periods different from the plurality of target sub-time periods in the scan time period; and (440) generating a target image sequence of the object (118) based on the plurality of target sets of scan data and the one or more intermediate images.

13. The method of claim 12, wherein the determining a plurality of target sets of scan data from the scan data based on a preset condition includes:

(510) dividing the scan data into a plurality of candidate sets of scan data; (520) for each of the plurality of candidate sets of scan data, determining a three-dimensional (3D) counting distribution map corresponding to the candidate set of scan data, wherein the 3D counting distribution map includes at least one pixel each of which corresponds to a pixel value indicating a count of coincidence events associated with the pixel; and (530) determining the plurality of target sets of scan data based on a plurality of 3D counting distribution maps corresponding to the plurality of target sets of scan data respectively.

14. The method of claim 13, wherein the determining the plurality of target sets of scan data based on a plurality of 3D counting distribution maps corresponding to the plurality of target sets of scan data respectively includes:

arranging the plurality of 3D counting distribution maps in chronological order to form a map sequence; and determining the plurality of target sets of scan data by traversing the map sequence starting from the first 3D counting distribution map in the map sequence, wherein the traversing the map sequence starting from the first 3D counting distribution map includes:
determining a difference between the latest 3D counting distribution map corresponding to the latest determined target set of scan data and each of the 3D counting distribution maps after the latest 3D counting distribution map in sequence until the difference between the latest 3D counting distribution map and one of the 3D counting distribution maps after the latest 3D counting distribution map is larger than or equal to a preset threshold, designating a candidate set of scan data corresponding to the one of the 3D counting distribution maps as a target set of scan data.

15. A non-transitory computer readable medium, comprising at least one set of instructions for positron emission tomography (PET) imaging, wherein when executed by one or more processors (210) of a computing device (200), the at least one set of instructions causes the computing device (200) to perform a method, the method comprising:

(410) obtaining scan data of an object (118) collected by a PET scan over a scan time period; **characterised by**
(420) determining a plurality of target sets of scan data from the scan data based on a preset condition, wherein each of the plurality of target sets of scan data corresponds to a target sub-time period in the scan time period;
(430) generating, based on the plurality of target sets of scan data, one or more intermediate images corresponding to one or more sub-time periods different from the plurality of target sub-time periods in the scan time period; and
(440) generating a target image sequence of the object (118) based on the plurality of target sets of scan data and the one or more intermediate images.

## Patentansprüche

1. System (100) zur Positronen-Emissions-Tomographie (PET)-Bildgebung, umfassend:

mindestens eine Speichervorrichtung (150), die einen Satz von Anleitungen für medizinische Bildgebung enthält; und
mindestens einen Prozessor (140), der in Kommunikation mit der mindestens einen Speichervorrichtung (150) steht, wobei der mindestens eine Prozessor (140) so konfiguriert ist, dass er, wenn er den Satz von Anweisungen ausführt, das System (100) anweist, Operationen durchzuführen, die Folgendes einschließen:

(410) Erhalten von Scandaten eines Objekts (118), die durch einen PET-Scan über einen Scanzeitraum gesammelt wurden; **gekennzeichnet durch**
(420) Bestimmen einer Vielzahl von Zielsätzen von Scandaten aus den Scandaten basierend auf einer voreingestellten Bedingung, wobei jeder der Vielzahl von Zielsätzen von Scandaten einem Zielteilzeitraum innerhalb des Scanzeitraums entspricht;
(430) Erzeugen, basierend auf der Vielzahl von Zielsätzen von Scandaten, eines oder mehrerer Zwischenbilder, die einem oder mehreren Teilzeiträumen entsprechen, die von der Vielzahl von Zielteilzeiträumen im Scanzeitraum unterschiedlich sind; und
(440) Erzeugen einer Zielbildsequenz des Objekts (118) basierend auf der Vielzahl von Zielsätzen von Scandaten und dem einen oder den mehrerer Zwischenbildern.

2. System (100) nach Anspruch 1, wobei das Bestimmen einer Vielzahl von Zielsätzen von Scandaten aus den Scandaten basierend auf einer voreingestellten Bedingung Folgendes einschließt:

(510) Aufteilen der Scandaten in eine Vielzahl von Kandidatensätzen von Scandaten;
(520) für jeden der Vielzahl von Kandidatensätze von Scandaten, Bestimmen einer dreidimensionalen (3D) Zählungsverteilungskarte, die dem Kandidatensatz von Scandaten entspricht, wobei die 3D-Zählungsverteilungskarte mindestens ein Pixel einschließt, von denen jedes einem Pixelwert entspricht, der eine Zählung von dem Pixel zugeordneten Übereinstimmungsereignissen angibt; und
(530) Bestimmen der Vielzahl von Zielsätzen von Scandaten basierend auf einer Vielzahl von 3D-Zählverteilungskarten, die jeweils der Vielzahl von Kandidatensätzen von Scandaten entspricht.

3. System (100) nach Anspruch 2, wobei das Bestimmen der Vielzahl von Zielsätzen von Scandaten basierend auf einer Vielzahl von 3D-Zählverteilungskarten, die jeweils der Vielzahl von Kandidatensätzen von Scandaten entspricht, Folgendes einschließt:

Anordnen der Vielzahl von 3D-Zählverteilungskarten in chronologischer Reihenfolge zum Bilden einer Kartensequenz; und

Bestimmen der Vielzahl von Zielsätzen von Scandaten durch Durchlaufen der Kartensequenz, beginnend mit der ersten 3D-Zählungsverteilungskarte in der Kartensequenz, wobei das Durchlaufen der Kartensequenz, beginnend mit der ersten 3D-Zählungsverteilungskarte, Folgendes einschließt:

Bestimmen einer Differenz zwischen der neuesten 3D-Zählungsverteilungskarte, die dem zuletzt bestimmten Zielsatz von Scandaten entspricht, und jeder der 3D-Zählverteilungskarten nach der neuesten 3D-Zählungsverteilungskarte in Folge, bis die Differenz zwischen der neuesten 3D-Zählungsverteilungskarte und einer der 3D-Zählverteilungskarten nach der neuesten 3D-Zählungsverteilungskarte größer oder gleich einem voreingestellten Schwellenwert ist, und Bezeichnen eines Kandidatensatzes von Scandaten, der der einen der 3D-Zählverteilungskarten entspricht, als Zielsatz von Scandaten.

4. System (100) nach Anspruch 1, wobei das Bestimmen einer Vielzahl von Zielsätzen von Scandaten aus den Scandaten basierend auf einer voreingestellten Bedingung Folgendes einschließt:

Erhalten einer Zeit-Aktivitäts-Kurve, die einem Tracer für den PET-Scan zugeordnet ist; und
Bestimmen der Vielzahl von Zielsätzen von Scandaten aus den Scandaten basierend auf der Zeit-Aktivitäts-Kurve.

5. System (100) nach Anspruch 1, wobei das Bestimmen einer Vielzahl von Zielsätzen von Scandaten aus den Scandaten basierend auf einer voreingestellten Bedingung Folgendes einschließt:

Erhalten eines Vitalzeichens des Objekts (118), das den Scandaten entspricht;
Bestimmen der Vielzahl von Zielsätzen von Scandaten aus den Scandaten basierend auf dem Vitalzeichen.

6. System (100) nach einem der Ansprüche 1-5, wobei das Erzeugen eines oder mehrerer Zwischenbilder, die einem oder mehreren Teilzeiträumen entsprechen, die von der Vielzahl von Zielteilzeiträumen im Scanzeitraum unterschiedlich sind, Folgendes einschließt:

Erzeugen einer Vielzahl von Zielbildern, die jeweils der Vielzahl von Zielsätzen von Scandaten entsprechen; und
Erzeugen eines oder mehrerer Zwischenbilder basierend auf der Vielzahl von Zielbildern.

7. System (100) nach Anspruch 6, wobei das Erzeugen einer Vielzahl von Zielbildern, die jeweils der Vielzahl von Zielsätzen von Scandaten entsprechen, Folgendes einschließt:

Bestimmen einer Vielzahl von vorläufigen Bildern, die jeweils der Vielzahl von Zielsätzen von Scandaten entsprechen; und
Bestimmen der Vielzahl von Zielbildern durch Durchführen einer Rauschunterdrückungsoperation an der Vielzahl von vorläufigen Bildern unter Verwendung eines Rauschunterdrückungsmodells und/oder Durchführen einer Auflösungsverbesserungsoperation an der Vielzahl von vorläufigen Bildern unter Verwendung eines Auflösungsverbesserungsmodells.

8. System (100) nach Anspruch 6 oder 7, wobei das Erzeugen eines oder mehrerer Zwischenbilder, die einem oder mehreren Teilzeiträumen entsprechen, die von der Vielzahl von Zielteilzeiträumen im Scanzeitraum unterschiedlich sind, Folgendes einschließt:
für jedes Paar von einem oder mehreren Paaren von Zielbildern aus der Vielzahl von Zielbildern,

Bestimmen eines Bewegungsfeldes zwischen dem Paar von Zielbildern unter Verwendung eines Bewegungsfelderzeugungsmodells; und
Erzeugen eines oder mehrerer Zwischenbilder, die dem Paar von Zielbildern entsprechen, basierend auf dem Bewegungsfeld unter Verwendung eines Bilderzeugungsmodells.

9. System (100) nach Anspruch 8, wobei das Bewegungsfelderzeugungsmodell und das Bilderzeugungsmodell in ein einziges Modell integriert sind.

10. System (100) nach einem der Ansprüche 6-9, wobei das Erzeugen einer Zielbildsequenz des Objekts (118) basierend auf der Vielzahl von Zielsätzen von Scandaten und dem einen oder den mehreren Zwischenbildern Folgendes einschließt:

für jedes Paar von einem oder mehreren Bildpaaren aus der Vielzahl von Zielbildern und dem einen oder den mehreren Zwischenbildern,

Bestimmen eines sekundären Bewegungsfeldes zwischen dem Paar von Bildern unter Verwendung eines Bewegungsfelderzeugungsmodells; und

Erzeugen eines oder mehrerer sekundärer Zwischenbilder, die dem Paar von Bildern entsprechen, basierend auf dem sekundären Bewegungsfeld unter Verwendung eines Bilderzeugungsmodells; und

Erzeugen der Zielbildsequenz des Objekts (118) basierend auf der Vielzahl von Zielbildern, dem einen oder den mehreren Zwischenbildern und dem einen oder den mehreren sekundären Zwischenbildern.

11. System (100) nach Anspruch 10, wobei eine Differenz zwischen dem Paar von Bildern größer ist als ein voreingestellter Differenzschwellenwert.

12. Verfahren zur Positronen-Emissions-Tomographie-(PET)-Bildgebung, wobei das Verfahren auf einer Computervorrichtung (200) implementiert ist, die mindestens eine Speichervorrichtung (220) und mindestens einen Prozessor (210) aufweist, wobei das Verfahren Folgendes umfasst:

(410) Erhalten von Scandaten eines Objekts (118), die durch einen PET-Scan über einen Scanzeitraum gesammelt wurden; **gekennzeichnet durch**

(420) Bestimmen einer Vielzahl von Zielsätzen von Scandaten aus den Scandaten basierend auf einer voreingestellten Bedingung, wobei jeder der Vielzahl von Zielsätzen von Scandaten einem Zielteilzeitraum innerhalb des Scanzeitraums entspricht;

(430) Erzeugen, basierend auf der Vielzahl von Zielsätzen von Scandaten, eines oder mehrerer Zwischenbilder, die einem oder mehreren Teilzeiträumen entsprechen, die von der Vielzahl von Zielteilzeiträumen im Scanzeitraum unterschiedlich sind; und

(440) Erzeugen einer Zielbildsequenz des Objekts (118) basierend auf der Vielzahl von Zielsätzen von Scandaten und dem einen oder den mehrerer Zwischenbildern.

13. Verfahren nach Anspruch 12, wobei das Bestimmen einer Vielzahl von Zielsätzen von Scandaten aus den Scandaten basierend auf einer voreingestellten Bedingung Folgendes einschließt:

(510) Aufteilen der Scandaten in eine Vielzahl von Kandidatensätzen von Scandaten;

(520) für jeden der Vielzahl von Kandidatensätze von Scandaten, Bestimmen einer dreidimensionalen (3D) Zählungsverteilungskarte, die dem Kandidatensatz von Scandaten entspricht, wobei die 3D-Zählungsverteilungskarte mindestens ein Pixel einschließt, von denen jedes einem Pixelwert entspricht, der eine Zählung von dem Pixel zugeordneten Übereinstimmungsereignissen angibt; und

(530) Bestimmen der Vielzahl von Zielsätzen von Scandaten basierend auf einer Vielzahl von 3D-Zählverteilungskarten, die jeweils der Vielzahl von Zielsätzen von Scandaten entspricht.

14. Verfahren nach Anspruch 13, wobei das Bestimmen der Vielzahl von Zielsätzen von Scandaten basierend auf einer Vielzahl von 3D-Zählverteilungskarten, die jeweils der Vielzahl von Zielsätzen von Scandaten entsprechen, Folgendes einschließt:

Anordnen der Vielzahl von 3D-Zählverteilungskarten in chronologischer Reihenfolge zum Bilden einer Kartensequenz; und

Bestimmen der Vielzahl von Zielsätzen von Scandaten durch Durchlaufen der Kartensequenz, beginnend mit der ersten 3D-Zählungsverteilungskarte in der Kartensequenz, wobei das Durchlaufen der Kartensequenz, beginnend mit der ersten 3D-Zählungsverteilungskarte, Folgendes einschließt:

Bestimmen einer Differenz zwischen der neuesten 3D-Zählungsverteilungskarte, die dem zuletzt bestimmten Zielsatz von Scandaten entspricht, und jeder der 3D-Zählverteilungskarten nach der neuesten 3D-Zählungsverteilungskarte in Folge, bis die Differenz zwischen der neuesten 3D-Zählungsverteilungskarte und einer der 3D-Zählverteilungskarten nach der neuesten 3D-Zählungsverteilungskarte größer oder gleich einem voreingestellten Schwellenwert ist, Bezeichnen eines Kandidatensatzes von Scandaten, der der einen der 3D-Zählverteilungskarten entspricht, als Zielsatz von Scandaten.

15. Nichtflüchtiges, computerlesbares Medium, das mindestens einen Satz von Anweisungen für Positronen-Emissions-Tomographie-(PET)-Bildgebung umfasst, wobei der mindestens eine Satz von Anweisungen, wenn er von einem oder mehreren Prozessoren (210) einer Rechenvorrichtung (200) ausgeführt wird, bewirkt, dass die Rechenvorrichtung (200) das Verfahren durchführt, wobei das Verfahren Folgendes umfasst:

(410) Erhalten von Scandaten eines Objekts (118), die durch einen PET-Scan über einen Scanzeitraum gesammelt wurden; **gekennzeichnet durch**

(420) Bestimmen einer Vielzahl von Zielsätzen von Scandaten aus den Scandaten basierend auf einer voreingestellten Bedingung, wobei jeder der Vielzahl von Zielsätzen von Scandaten einem Zielteilzeitraum innerhalb des Scanzeitraums entspricht;

(430) Erzeugen, basierend auf der Vielzahl von Zielsätzen von Scandaten, eines oder mehrerer Zwischenbilder, die einem oder mehreren Teilzeiträumen entsprechen, die von der Vielzahl von Zielteilzeiträumen im Scanzeitraum unterschiedlich sind; und

(440) Erzeugen einer Zielbildsequenz des Objekts (118) basierend auf der Vielzahl von Zielsätzen von Scandaten und dem einen oder den mehrerer Zwischenbildern.

**Revendications**

1. Système (100) d'imagerie par tomographie à émission de positrons (PET), comprenant :

au moins un dispositif de stockage (150) incluant un ensemble d'instructions pour l'imagerie médicale ; et
au moins un processeur (140) en communication avec le au moins un dispositif de stockage (150), dans lequel lors de l'exécution de l'ensemble d'instructions, le au moins un processeur (140) est configuré pour amener le système (100) à effectuer des opérations incluant :

(410) l'obtention de données de balayage d'un objet (118) recueillies par un balayage PET sur une période de temps de balayage ; **caractérisé par**

(420) la détermination d'une pluralité d'ensembles cibles de données de balayage à partir des données de balayage sur la base d'une condition prédéfinie, dans lequel chacun de la pluralité d'ensembles cibles de données de balayage correspond à une sous-période cible dans la période de balayage ;

(430) la génération, sur la base de la pluralité d'ensembles cibles de données de balayage, d'une ou plusieurs images intermédiaires correspondant à une ou plusieurs sous-périodes différentes de la pluralité de sous-périodes cibles dans la période de balayage ; et

(440) la génération d'une séquence d'images cibles de l'objet (118) sur la base de la pluralité d'ensembles cibles de données de balayage et des une ou plusieurs images intermédiaires.

2. Système (100) selon la revendication 1, dans lequel la détermination d'une pluralité d'ensembles cibles de données de balayage à partir des données de balayage sur la base d'une condition prédéfinie inclut :

(510) la division des données de balayage en une pluralité d'ensembles candidats de données de balayage ;

(520) pour chacun de la pluralité d'ensembles candidats de données de balayage, la détermination d'une carte de distribution de comptage tridimensionnelle (3D) correspondant à l'ensemble candidat de données de balayage, dans lequel la carte de distribution de comptage 3D inclut au moins un pixel, dont chacun correspond à une valeur de pixel indiquant un nombre d'événements de coïncidence associés au pixel ; et

(530) la détermination de la pluralité d'ensembles cibles de données de balayage sur la base d'une pluralité de cartes de distribution de comptage 3D correspondant respectivement à la pluralité d'ensembles candidats de données de balayage.

3. Système (100) selon la revendication 2, dans lequel la détermination de la pluralité d'ensembles cibles de données de balayage sur la base d'une pluralité de cartes de distribution de comptage 3D correspondant respectivement à la pluralité d'ensembles candidats de données de balayage inclut :

l'agencement de la pluralité de cartes de distribution de comptage 3D par ordre chronologique pour former une séquence de cartes ; et
la détermination de la pluralité d'ensembles cibles de données de balayage en parcourant la séquence de cartes à partir de la première carte de distribution de comptage 3D de la séquence de cartes, dans lequel le parcours de la séquence de cartes à partir de la première carte de distribution de comptage 3D inclut :
la détermination d'une différence entre la dernière carte de distribution de comptage 3D correspondant au dernier ensemble cible de données de balayage déterminé et chacune des cartes de distribution de comptage 3D après la dernière carte de distribution de comptage 3D en séquence jusqu'à ce que la différence entre la dernière carte de distribution de comptage 3D et l'une des cartes de distribution de comptage 3D après la dernière carte de distribution de comptage 3D soit supérieure ou égale à un seuil prédéfini, et la désignation d'un ensemble candidat de données de balayage correspondant à l'une des cartes de distribution de comptage 3D comme ensemble cible de données de balayage.

4. Système (100) selon la revendication 1, dans lequel la détermination d'une pluralité d'ensembles cibles de données de balayage à partir des données de balayage sur la base d'une condition prédéfinie inclut :

l'obtention d'une courbe d'activité temporelle associée à un traceur pour le balayage PET ; et
la détermination de la pluralité d'ensembles cibles de données de balayage à partir des données de balayage en fonction de la courbe d'activité temporelle.

5. Système (100) selon la revendication 1, dans lequel la détermination d'une pluralité d'ensembles cibles de données de balayage à partir des données de balayage sur la base d'une condition prédéfinie inclut :

l'obtention d'un signal vital de l'objet (118) correspondant aux données de balayage ;
la détermination de la pluralité d'ensembles cibles de données de balayage à partir des données de balayage sur la base du signal vital.

6. Système (100) selon l'une quelconque des revendications 1-5, dans lequel la génération d'une ou plusieurs images intermédiaires correspondant à une ou plusieurs sous-périodes différentes de la pluralité de sous-périodes cibles dans la période de balayage, inclut :

la génération d'une pluralité d'images cibles correspondant respectivement à la pluralité d'ensembles cibles de données de balayage ; et
la génération des une ou plusieurs images intermédiaires sur la base de la pluralité d'images cibles.

7. Système (100) selon la revendication 6, dans lequel la génération d'une pluralité d'images cibles correspondant à la pluralité d'ensembles cibles de données de balayage inclut respectivement :

la détermination d'une pluralité d'images préliminaires correspondant respectivement à la pluralité d'ensembles cibles de données de balayage ; et
la détermination de la pluralité d'images cibles en effectuant une opération de débruitage sur la pluralité d'images préliminaires à l'aide d'un modèle de débruitage et/ou en effectuant une opération d'amélioration de la résolution sur la pluralité d'images préliminaires à l'aide d'un modèle d'amélioration de la résolution.

8. Système (100) selon la revendication 6 ou 7, dans lequel la génération d'une ou plusieurs images intermédiaires correspondant à une ou plusieurs sous-périodes différentes de la pluralité de sous-périodes cibles dans la période de balayage inclut :
pour chaque paire d'une ou plusieurs paires d'images cibles parmi la pluralité d'images cibles,

la détermination d'un champ de mouvement entre la paire d'images cibles à l'aide d'un modèle de génération de champ de mouvement ; et
la génération d'une ou plusieurs images intermédiaires correspondant à la paire d'images cibles sur la base du champ de mouvement à l'aide d'un modèle de génération d'images.

9. Système (100) selon la revendication 8, dans lequel le modèle de génération de champ de mouvement et le modèle de génération d'image sont intégrés dans un seul modèle.

10. Système (100) selon l'une quelconque des revendications 6-9, dans lequel la génération d'une séquence d'images cibles de l'objet (118) sur la base de la pluralité d'ensembles cibles de données de balayage et des une ou plusieurs images intermédiaires inclut :

pour chaque paire d'une ou plusieurs paires d'images parmi la pluralité d'images cibles et les une ou plusieurs images intermédiaires,

la détermination d'un champ de mouvement secondaire entre la paire d'images à l'aide d'un modèle de génération de champ de mouvement ; et

la génération d'une ou plusieurs images intermédiaires secondaires correspondant à la paire d'images sur la base du champ de mouvement secondaire à l'aide d'un modèle de génération d'images ; et

la génération de la séquence d'images cibles de l'objet (118) sur la base de la pluralité d'images cibles, des une ou plusieurs images intermédiaires, des une ou plusieurs images intermédiaires secondaires.

11. Système (100) selon la revendication 10, dans lequel une différence entre la paire d'images est supérieure à un seuil de différence prédéfini.

12. Procédé d'imagerie par tomographie à émission de positrons (PET), le procédé étant mis en œuvre sur un dispositif informatique (200) présentant au moins un dispositif de stockage (220) et au moins un processeur (210), le procédé comprenant :

(410) l'obtention de données de balayage d'un objet (118) recueillies par un balayage PET sur une période de balayage ; **caractérisé par**

(420) la détermination d'une pluralité d'ensembles cibles de données de balayage à partir des données de balayage sur la base d'une condition prédéfinie, dans lequel chacun de la pluralité d'ensembles cibles de données de balayage correspond à une sous-période cible dans la période de balayage ;

(430) la génération, à partir de la pluralité d'ensembles cibles de données de balayage, d'une ou plusieurs images intermédiaires correspondant à une ou plusieurs sous-périodes différentes de la pluralité de sous-périodes cibles dans la période de balayage ; et

(440) la génération d'une séquence d'images cibles de l'objet (118) sur la base de la pluralité d'ensembles cibles de données de balayage et des une ou plusieurs images intermédiaires.

13. Procédé selon la revendication 12, dans lequel la détermination d'une pluralité d'ensembles cibles de données de balayage à partir des données de balayage, sur la base d'une condition prédéfinie, inclut :

(510) la division des données de balayage en une pluralité d'ensembles candidats de données de balayage ;

(520) pour chacun de la pluralité d'ensembles candidats de données de balayage, la détermination d'une carte de distribution de comptage tridimensionnelle (3D) correspondant à l'ensemble candidat de données de balayage, dans lequel la carte de distribution de comptage 3D inclut au moins un pixel dont chacun correspond à une valeur de pixel indiquant le nombre d'événements de coïncidence associés au pixel ; et

(530) la détermination de la pluralité d'ensembles cibles de données de balayage sur la base d'une pluralité de cartes de distribution de comptage 3D correspondant respectivement à la pluralité d'ensembles cibles de données de balayage.

14. Procédé selon la revendication 13, dans lequel la détermination de la pluralité d'ensembles cibles de données de balayage sur la base d'une pluralité de cartes de distribution de comptage 3D correspondant respectivement à la pluralité d'ensembles cibles de données de balayage inclut :

l'agencement de la pluralité de cartes de distribution de comptage 3D par ordre chronologique pour former une séquence de cartes ; et

la détermination de la pluralité d'ensembles cibles de données de balayage en parcourant la séquence de cartes à partir de la première carte de distribution de comptage 3D de la séquence de cartes, dans lequel le parcours de la séquence de cartes à partir de la première carte de distribution de comptage 3D inclut :

la détermination d'une différence entre la dernière carte de distribution de comptage 3D correspondant au dernier ensemble cible de données de balayage déterminé et chacune des cartes de distribution de comptage 3D après la dernière carte de distribution de comptage 3D en séquence jusqu'à ce que la différence entre la dernière carte de distribution de comptage 3D et l'une des cartes de distribution de comptage 3D après la dernière carte de distribution de comptage 3D soit supérieure ou égale à un seuil prédéfini, la désignation d'un ensemble candidat de données de balayage correspondant à l'une des cartes de distribution de comptage 3D comme ensemble cible de données de balayage.

15. Support non transitoire lisible par ordinateur, comprenant au moins un ensemble d'instructions pour une imagerie par tomographie à émission de positrons, dans lequel lorsqu'il est exécuté par un ou plusieurs processeurs (210) d'un dispositif informatique (200), l'au moins un ensemble d'instructions amène le dispositif informatique (200) à mettre en œuvre un procédé, le procédé comprenant :

(410) l'obtention de données de balayage d'un objet (118) recueillies par un balayage PET sur une période de balayage ; **caractérisé par**

(420) la détermination d'une pluralité d'ensembles cibles de données de balayage à partir des données de balayage sur la base d'une condition prédéfinie, dans lequel chacun de la pluralité d'ensembles cibles de données de balayage correspond à une sous-période cible dans la période de balayage ;

(430) la génération, à partir de la pluralité d'ensembles cibles de données de balayage, d'une ou plusieurs images intermédiaires correspondant à une ou plusieurs sous-périodes différentes de la pluralité de sous-périodes cibles dans la période de balayage ; et

(440) la génération d'une séquence d'images cibles de l'objet (118) sur la base de la pluralité d'ensembles cibles de données de balayage et des une ou plusieurs images intermédiaires.

**100**

**FIG. 1**

**200**

210

Processor

220

Storage

230

I/O

240

Communication port

**FIG. 2**

**140**

Obtaining Module

310

Determination Module

320

Generation Module

330

**FIG. 3**

**400**

```
┌─────────────────────────────────────────────────────┐
│  Obtaining scan data of an object collected by a PET  │      410
│  scan over a scan time period                         │
└─────────────────────────────────────────────────────┘
                          │
                          ▼
┌─────────────────────────────────────────────────────┐
│  Determining a plurality of target sets of scan data  │      420
│  from the scan data based on a preset condition,      │
│  wherein each of the plurality of target sets of scan │
│  data corresponds to a target sub-time period in the  │
│  scan time period                                     │
└─────────────────────────────────────────────────────┘
                          │
                          ▼
┌─────────────────────────────────────────────────────┐
│  Generating, based on the plurality of target sets of │      430
│  scan data, one or more intermediate images           │
│  corresponding to one or more sub-time periods        │
│  different from the plurality of target sub-time       │
│  periods in the scan time period                      │
└─────────────────────────────────────────────────────┘
                          │
                          ▼
┌─────────────────────────────────────────────────────┐
│  Generating a target image sequence of the object     │      440
│  based the plurality of target sets of scan data and  │
│  the one or more intermediate images                  │
└─────────────────────────────────────────────────────┘
```

**FIG. 4**

**500**

| Dividing the scan data into a plurality of candidate sets of scan data | 510 |

↓

| For each of the plurality of candidate sets of scan data, determining a three-dimensional (3D) counting distribution map corresponding to the candidate set of scan data | 520 |

↓

| Determining the plurality of target sets of scan data based on a plurality of 3D counting distribution maps corresponding to the plurality of candidate sets of scan data respectively | 530 |

**FIG. 5A**

501 502 503 504 505 506 507 508 509

**FIG. 5B**

Concentration of tracer

**FIG. 6**

Respiratory amplitude

**FIG. 7**

FIG. 8

<u>900</u>

FIG. 9

**1000A**

FIG. 10A

**1000B**

FIG. 10B

**EP 4 510 935 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013261440 A1